# EUROPEAN PATENT APPLICATION

(11) **EP 1 669 446 A1**
(43) Date of publication of application: **14.06.2006**
(21) Application number: 04773069.2
(22) Date of filing: 08.09.2004
(51) Int. Cl.: C12N 15/09, C12Q 1/02, C12Q 1/68, G01N 33/15, G01N 33/50, A61P 9/10

(54) **SCREENING METHOD**

(30) Priority: 09.09.2003 JP 2003316572
(71) Applicant: Takeda Pharmaceutical Company Limited, Osaka-shi, Osaka 541-0045 (JP)
(72) Inventor: HAYASHI, Ryogo, Ikeda-shi, Osaka 5630022 (JP); IIZAWA, Yuji, Muko-shi, Kyoto 6170002 (JP)
(74) Representative: Lewin, John Harvey
(86) International application number: PCT/JP2004/013396
(87) International publication number: WO 2005/026348

(57) **Abstract**

The present invention provides a screening means for a novel therapeutic drug for arteriosclerosis capable of suppressing the progression of arteriosclerosis due to infection with *Chlamydia pneumoniae*. Specifically, provided is a screening method for a therapeutic substance for arteriosclerosis and/or a.prophylactic/therapeutic substance for complications thereof, which comprises using cells of an established monocytic cell line and a *Chlamydia pneumoniae-*derived 60-kDa heat shock protein or a partial peptide thereof or a salt thereof or calls capable of producing the same (the cells may be identical to, or different from, the cells of the established monocytic cell line).

SEQUENCE LISTING

<110>  Takeda Pharmaceutical Company Limited

<120>  Screening Method

<130>  1464

<150>  JP2003-316572
<151>  2003-09-09

<160>  6

<170>  PatentIn version 3.1

<210>  1
<211>  1632
<212>  DNA
<213>  Chlamydia pneumoniae strain KKpn-1

<220>
<221>  CDS
<222>  (1)..(1632)
<223>

<400>  1
atg gca gcg aaa aat att aaa tat aat gaa gaa gcc aga aaa aaa ata        48
Met Ala Ala Lys Asn Ile Lys Tyr Asn Glu Glu Ala Arg Lys Lys Ile
1               5                   10                  15
cat aaa ggg gta aaa act ctt gca gaa gca gta aaa gtt act cta ggt        96
His Lys Gly Val Lys Thr Leu Ala Glu Ala Val Lys Val Thr Leu Gly
            20                  25                  30
cct aaa gga cgt cac gta gtt ata gat aag agc ttt ggc tct ccc caa        144
Pro Lys Gly Arg His Val Val Ile Asp Lys Ser Phe Gly Ser Pro Gln
        35                  40                  45
gtg act aaa gat ggt gtt act gta gct aaa gaa atc gag ctc gaa gac        192
Val Thr Lys Asp Gly Val Thr Val Ala Lys Glu Ile Glu Leu Glu Asp
    50                  55                  60
aaa cat gaa aac atg ggc gct cag atg gta aaa gaa gtc gcc agc aaa        240
Lys His Glu Asn Met Gly Ala Gln Met Val Lys Glu Val Ala Ser Lys
65                  70                  75                  80
act gct gac aaa gca ggc gac gga act aca aca gca act gtt ctt gca        288
Thr Ala Asp Lys Ala Gly Asp Gly Thr Thr Thr Ala Thr Val Leu Ala
                85                  90                  95
gaa gca atc tat agc gaa ggt cta aga aat gtc act gcc ggt gcc aat        336
Glu Ala Ile Tyr Ser Glu Gly Leu Arg Asn Val Thr Ala Gly Ala Asn
            100                 105                 110
cct atg gac cta aaa aga ggt atc gac aaa gcc gta aaa gtt gtt gtt        384
Pro Met Asp Leu Lys Arg Gly Ile Asp Lys Ala Val Lys Val Val Val
        115                 120                 125
gat gaa ctc aaa aaa att agt aaa cct gta caa cat cac aaa gaa atc        432
Asp Glu Leu Lys Lys Ile Ser Lys Pro Val Gln His His Lys Glu Ile
    130                 135                 140
gct caa gta gct act atc tca gca aat aat gat tcc gaa atc gga aat        480
Ala Gln Val Ala Thr Ile Ser Ala Asn Asn Asp Ser Glu Ile Gly Asn
145                 150                 155                 160
ctt att gca gaa gct atg gaa aaa gtt ggt aaa aac gga tcc att act        528
Leu Ile Ala Glu Ala Met Glu Lys Val Gly Lys Asn Gly Ser Ile Thr
                165                 170                 175
gtt gaa gaa gct aaa ggc ttc gaa act gtt ctc gac gtt gta gaa gga        576
Val Glu Glu Ala Lys Gly Phe Glu Thr Val Leu Asp Val Val Glu Gly
            180                 185                 190
atg aac ttc aac cgt gga tac ctc tcc agc tac ttc tcc aca aat cca        624
Met Asn Phe Asn Arg Gly Tyr Leu Ser Ser Tyr Phe Ser Thr Asn Pro
        195                 200                 205
gaa act caa gaa tgc gtt tta gaa gac gct ctg att cta atc tac gat        672
Glu Thr Gln Glu Cys Val Leu Glu Asp Ala Leu Ile Leu Ile Tyr Asp
    210                 215                 220
aaa aaa atc tct gga att aaa gac ttc ctt cca gtt tta caa caa gta        720
Lys Lys Ile Ser Gly Ile Lys Asp Phe Leu Pro Val Leu Gln Gln Val
225                 230                 235                 240
gca gaa tct gga cgc cct ctt tta atc att gca gaa gaa att gaa gga        768
Ala Glu Ser Gly Arg Pro Leu Leu Ile Ile Ala Glu Glu Ile Glu Gly
                245                 250                 255
gaa gct tta gca act cta gta gtc aat aga ctc cgt gca gga ttc aga        816
Glu Ala Leu Ala Thr Leu Val Val Asn Arg Leu Arg Ala Gly Phe Arg
            260                 265                 270

gtc tgt gca gtg aaa gct cct ggt ttc ggt gac aga aga aaa gct atg 864
Val Cys Ala Val Lys Ala Pro Gly Phe Gly Asp Arg Arg Lys Ala Met
        275                 280                 285
tta gaa gac atc gct atc ctt act ggt ggc caa cta gtt agc gaa gaa 912
Leu Glu Asp Ile Ala Ile Leu Thr Gly Gly Gln Leu Val Ser Glu Glu
    290                 295                 300
ctt ggc atg aaa cta gag aat aca act cta gca atg tta gga aaa gct 960
Leu Gly Met Lys Leu Glu Asn Thr Thr Leu Ala Met Leu Gly Lys Ala
305                 310                 315                 320
aag aaa gtt atc gta act aaa gaa gat acc aca atc gtc gaa ggc tta 1008
Lys Lys Val Ile Val Thr Lys Glu Asp Thr Thr Ile Val Glu Gly Leu
                325                 330                 335
gga aac aaa cct gat atc caa gct cga tgc gac aat att aaa aaa caa 1056
Gly Asn Lys Pro Asp Ile Gln Ala Arg Cys Asp Asn Ile Lys Lys Gln
            340                 345                 350
atc gaa gat agc act tca gat tac gac aaa gaa aaa ctc caa gag cgt 1104
Ile Glu Asp Ser Thr Ser Asp Tyr Asp Lys Glu Lys Leu Gln Glu Arg
        355                 360                 365
tta gct aaa ctc tcc ggt ggt gtc gcc gta atc cgc gta gga gct gct 1152
Leu Ala Lys Leu Ser Gly Gly Val Ala Val Ile Arg Val Gly Ala Ala
    370                 375                 380
acc gaa ata gag atg aaa gag aaa aaa gac aga gta gat gat gca caa 1200
Thr Glu Ile Glu Met Lys Glu Lys Lys Asp Arg Val Asp Asp Ala Gln
385                 390                 395                 400
cac gca acc att gca gct gtc gaa gaa gga atc ctc cct ggt ggt gga 1248
His Ala Thr Ile Ala Ala Val Glu Glu Gly Ile Leu Pro Gly Gly Gly
                405                 410                 415
act gcc tta gtt cgc tgt atc cct aca cta gaa gct ttc ctt cct atg 1296
Thr Ala Leu Val Arg Cys Ile Pro Thr Leu Glu Ala Phe Leu Pro Met
            420                 425                 430
cta gca aac gaa gac gaa gct att ggt act cgt att att cta aaa gca 1344
Leu Ala Asn Glu Asp Glu Ala Ile Gly Thr Arg Ile Ile Leu Lys Ala
        435                 440                 445
tta aca gct cca tta aag caa att gca agt aac gca ggt aaa gaa ggc 1392
Leu Thr Ala Pro Leu Lys Gln Ile Ala Ser Asn Ala Gly Lys Glu Gly
    450                 455                 460
gct atc att tgt cag caa gtt cta gca aga tct gca aat gaa ggc tat 1440
Ala Ile Ile Cys Gln Gln Val Leu Ala Arg Ser Ala Asn Glu Gly Tyr
465                 470                 475                 480
gat gct tta cgt gac gct tat aca gat atg att gac gca gga att tta 1488
Asp Ala Leu Arg Asp Ala Tyr Thr Asp Met Ile Asp Ala Gly Ile Leu
                485                 490                 495
gat cca act aaa gtg act cgc tca gct cta gaa agc gca gct tct atc 1536
Asp Pro Thr Lys Val Thr Arg Ser Ala Leu Glu Ser Ala Ala Ser Ile
            500                 505                 510
gca gga tta ctc ctc aca aca gaa gcc tta atc gct gat atc cca gaa 1584
Ala Gly Leu Leu Leu Thr Thr Glu Ala Leu Ile Ala Asp Ile Pro Glu
        515                 520                 525
gag aaa tct tct tca gct cca gcg atg cca agc gca gga atg gac tac 1632
Glu Lys Ser Ser Ser Ala Pro Ala Met Pro Ser Ala Gly Met Asp Tyr
    530                 535                 540

<210> 2
<211> 544
<212> PRT
<213> Chlamydia pneumoniae strain KKpn-1

<400> 2
Met Ala Ala Lys Asn Ile Lys Tyr Asn Glu Glu Ala Arg Lys Lys Ile
1               5                   10                  15
His Lys Gly Val Lys Thr Leu Ala Glu Ala Val Lys Val Thr Leu Gly
            20                  25                  30
Pro Lys Gly Arg His Val Val Ile Asp Lys Ser Phe Gly Ser Pro Gln
        35                  40                  45
Val Thr Lys Asp Gly Val Thr Val Ala Lys Glu Ile Glu Leu Glu Asp
    50                  55                  60
Lys His Glu Asn Met Gly Ala Gln Met Val Lys Glu Val Ala Ser Lys
65                  70                  75                  80
Thr Ala Asp Lys Ala Gly Asp Gly Thr Thr Thr Ala Thr Val Leu Ala
                85                  90                  95
Glu Ala Ile Tyr Ser Glu Gly Leu Arg Asn Val Thr Ala Gly Ala Asn
            100                 105                 110
Pro Met Asp Leu Lys Arg Gly Ile Asp Lys Ala Val Lys Val Val Val
        115                 120                 125
Asp Glu Leu Lys Lys Ile Ser Lys Pro Val Gln His His Lys Glu Ile
    130                 135                 140
Ala Gln Val Ala Thr Ile Ser Ala Asn Asn Asp Ser Glu Ile Gly Asn

145                 150                 155                 160
Leu Ile Ala Glu Ala Met Glu Lys Val Gly Lys Asn Gly Ser Ile Thr
                165                 170                 175
Val Glu Glu Ala Lys Gly Phe Glu Thr Val Leu Asp Val Val Glu Gly
            180                 185                 190
Met Asn Phe Asn Arg Gly Tyr Leu Ser Ser Tyr Phe Ser Thr Asn Pro
        195                 200                 205
Glu Thr Gln Glu Cys Val Leu Glu Asp Ala Leu Ile Leu Ile Tyr Asp
    210                 215                 220
Lys Lys Ile Ser Gly Ile Lys Asp Phe Leu Pro Val Leu Gln Gln Val
225                 230                 235                 240
Ala Glu Ser Gly Arg Pro Leu Leu Ile Ile Ala Glu Glu Ile Glu Gly
                245                 250                 255
Glu Ala Leu Ala Thr Leu Val Val Asn Arg Leu Arg Ala Gly Phe Arg
            260                 265                 270
Val Cys Ala Val Lys Ala Pro Gly Phe Gly Asp Arg Arg Lys Ala Met
        275                 280                 285
Leu Glu Asp Ile Ala Ile Leu Thr Gly Gly Gln Leu Val Ser Glu Glu
    290                 295                 300
Leu Gly Met Lys Leu Glu Asn Thr Thr Leu Ala Met Leu Gly Lys Ala
305                 310                 315                 320
Lys Lys Val Ile Val Thr Lys Glu Asp Thr Thr Ile Val Glu Gly Leu
                325                 330                 335
Gly Asn Lys Pro Asp Ile Gln Ala Arg Cys Asp Asn Ile Lys Lys Gln
            340                 345                 350
Ile Glu Asp Ser Thr Ser Asp Tyr Asp Lys Glu Lys Leu Gln Glu Arg
        355                 360                 365
Leu Ala Lys Leu Ser Gly Gly Val Ala Val Ile Arg Val Gly Ala Ala
    370                 375                 380
Thr Glu Ile Glu Met Lys Glu Lys Lys Asp Arg Val Asp Asp Ala Gln
385                 390                 395                 400
His Ala Thr Ile Ala Ala Val Glu Glu Gly Ile Leu Pro Gly Gly Gly
                405                 410                 415
Thr Ala Leu Val Arg Cys Ile Pro Thr Leu Glu Ala Phe Leu Pro Met
            420                 425                 430
Leu Ala Asn Glu Asp Glu Ala Ile Gly Thr Arg Ile Ile Leu Lys Ala
        435                 440                 445
Leu Thr Ala Pro Leu Lys Gln Ile Ala Ser Asn Ala Gly Lys Glu Gly
    450                 455                 460
Ala Ile Ile Cys Gln Gln Val Leu Ala Arg Ser Ala Asn Glu Gly Tyr
465                 470                 475                 480
Asp Ala Leu Arg Asp Ala Tyr Thr Asp Met Ile Asp Ala Gly Ile Leu
                485                 490                 495
Asp Pro Thr Lys Val Thr Arg Ser Ala Leu Glu Ser Ala Ala Ser Ile
            500                 505                 510
Ala Gly Leu Leu Leu Thr Thr Glu Ala Leu Ile Ala Asp Ile Pro Glu
        515                 520                 525
Glu Lys Ser Ser Ser Ala Pro Ala Met Pro Ser Ala Gly Met Asp Tyr
    530                 535                 540

<210>  3
<211>  2121
<212>  DNA
<213>  Homo sapiens

<220>
<221>  CDS
<222>  (1)..(2121)
<223>

<220>
<221>  sig_peptide
<222>  (1)..(57)
<223>

<220>
<221>  mat_peptide
<222>  (319)..(2121)
<223>

<400>  3
atg agc ctc tgg cag ccc ctg gtc ctg gtg ctc ctg gtg ctg ggc tgc      48
Met Ser Leu Trp Gln Pro Leu Val Leu Val Leu Leu Val Leu Gly Cys
    -105                -100                -95
tgc ttt gct gcc ccc aga cag cgc cag tcc acc ctt gtg ctc ttc cct      96
Cys Phe Ala Ala Pro Arg Gln Arg Gln Ser Thr Leu Val Leu Phe Pro
-90                 -85                 -80                 -75

gga gac ctg aga acc aat ctc acc gac agg cag ctg gca gag gaa tac 144
Gly Asp Leu Arg Thr Asn Leu Thr Asp Arg Gln Leu Ala Glu Glu Tyr
-70 -65 -60
ctg tac cgc tat ggt tac act cgg gtg gca gag atg cgt gga gag tcg 192
Leu Tyr Arg Tyr Gly Tyr Thr Arg Val Ala Glu Met Arg Gly Glu Ser
-55 -50 -45
aaa tct ctg ggg cct gcg ctg ctg ctt ctc cag aag caa ctg tcc ctg 240
Lys Ser Leu Gly Pro Ala Leu Leu Leu Leu Gln Lys Gln Leu Ser Leu
-40 -35 -30
ccc gag acc ggt gag ctg gat agc gcc acg ctg aag gcc atg cga acc 288
Pro Glu Thr Gly Glu Leu Asp Ser Ala Thr Leu Lys Ala Met Arg Thr
-25 -20 -15
cca cgg tgc ggg gtc cca gac ctg ggc aga ttc caa acc ttt gag ggc 336
Pro Arg Cys Gly Val Pro Asp Leu Gly Arg Phe Gln Thr Phe Glu Gly
-10 -5 -1 1 5
gac ctc aag tgg cac cac cac aac atc acc tat tgg atc caa aac tac 384
Asp Leu Lys Trp His His His Asn Ile Thr Tyr Trp Ile Gln Asn Tyr
10 15 20
tcg gaa gac ttg ccg cgg gcg gtg att gac gac gcc ttt gcc cgc gcc 432
Ser Glu Asp Leu Pro Arg Ala Val Ile Asp Asp Ala Phe Ala Arg Ala
25 30 35
ttc gca ctg tgg agc gcg gtg acg ccg ctc acc ttc act cgc gtg tac 480
Phe Ala Leu Trp Ser Ala Val Thr Pro Leu Thr Phe Thr Arg Val Tyr
40 45 50
agc cgg gac gca gac atc gtc atc cag ttt ggt gtc gcg gag cac gga 528
Ser Arg Asp Ala Asp Ile Val Ile Gln Phe Gly Val Ala Glu His Gly
55 60 65 70
gac ggg tat ccc ttc gac ggg aag gac ggg ctc ctg gca cac gcc ttt 576
Asp Gly Tyr Pro Phe Asp Gly Lys Asp Gly Leu Leu Ala His Ala Phe
75 80 85
cct cct ggc ccc ggc att cag gga gac gcc cat ttc gac gat gac gag 624
Pro Pro Gly Pro Gly Ile Gln Gly Asp Ala His Phe Asp Asp Asp Glu
90 95 100
ttg tgg tcc ctg ggc aag ggc gtc gtg gtt cca act cgg ttt gga aac 672
Leu Trp Ser Leu Gly Lys Gly Val Val Val Pro Thr Arg Phe Gly Asn
105 110 115
gca gat ggc gcg gcc tgc cac ttc ccc ttc atc ttc gag ggc cgc tcc 720
Ala Asp Gly Ala Ala Cys His Phe Pro Phe Ile Phe Glu Gly Arg Ser
120 125 130
tac tct gcc tgc acc acc gac ggt cgc tcc gac ggc ttg ccc tgg tgc 768
Tyr Ser Ala Cys Thr Thr Asp Gly Arg Ser Asp Gly Leu Pro Trp Cys
135 140 145 150
agt acc acg gcc aac tac gac acc gac gac cgg ttt ggc ttc tgc ccc 816
Ser Thr Thr Ala Asn Tyr Asp Thr Asp Asp Arg Phe Gly Phe Cys Pro
155 160 165
agc gag aga ctc tac acc cgg gac ggc aat gct gat ggg aaa ccc tgc 864
Ser Glu Arg Leu Tyr Thr Arg Asp Gly Asn Ala Asp Gly Lys Pro Cys
170 175 180
cag ttt cca ttc atc ttc caa ggc caa tcc tac tcc gcc tgc acc acg 912
Gln Phe Pro Phe Ile Phe Gln Gly Gln Ser Tyr Ser Ala Cys Thr Thr
185 190 195
gac ggt cgc tcc gac ggc tac cgc tgg tgc gcc acc acc gcc aac tac 960
Asp Gly Arg Ser Asp Gly Tyr Arg Trp Cys Ala Thr Thr Ala Asn Tyr
200 205 210
gac cgg gac aag ctc ttc ggc ttc tgc ccg acc cga gct gac tcg acg 1008
Asp Arg Asp Lys Leu Phe Gly Phe Cys Pro Thr Arg Ala Asp Ser Thr
215 220 225 230
gtg atg ggg ggc aac tcg gcg ggg gag ctg tgc gtc ttc ccc ttc act 1056
Val Met Gly Gly Asn Ser Ala Gly Glu Leu Cys Val Phe Pro Phe Thr
235 240 245
ttc ctg ggt aag gag tac tcg acc tgt acc agc gag ggc cgc gga gat 1104
Phe Leu Gly Lys Glu Tyr Ser Thr Cys Thr Ser Glu Gly Arg Gly Asp
250 255 260
ggg cgc ctc tgg tgc gct acc acc tcg aac ttt gac agc gac aag aag 1152
Gly Arg Leu Trp Cys Ala Thr Thr Ser Asn Phe Asp Ser Asp Lys Lys
265 270 275
tgg ggc ttc tgc ccg gac caa gga tac agt ttg ttc ctc gtg gcg gcg 1200
Trp Gly Phe Cys Pro Asp Gln Gly Tyr Ser Leu Phe Leu Val Ala Ala
280 285 290
cat gag ttc ggc cac gcg ctg ggc tta gat cat tcc tca gtg ccg gag 1248
His Glu Phe Gly His Ala Leu Gly Leu Asp His Ser Ser Val Pro Glu
295 300 305 310
gcg ctc atg tac cct atg tac cgc ttc act gag ggg ccc ccc ttg cat 1296
Ala Leu Met Tyr Pro Met Tyr Arg Phe Thr Glu Gly Pro Pro Leu His
315 320 325
aag gac gac gtg aat ggc atc cgg cac ctc tat ggt cct cgc cct gaa 1344
Lys Asp Asp Val Asn Gly Ile Arg His Leu Tyr Gly Pro Arg Pro Glu

330 335 340
cct gag cca cgg cct cca acc acc acc aca ccg cag ccc acg gct ccc 1392
Pro Glu Pro Arg Pro Pro Thr Thr Thr Thr Pro Gln Pro Thr Ala Pro
345 350 355
ccg acg gtc tgc ccc acc gga ccc ccc act gtc cac ccc tca gag cgc 1440
Pro Thr Val Cys Pro Thr Gly Pro Pro Thr Val His Pro Ser Glu Arg
360 365 370
ccc aca gct ggc ccc aca ggt ccc ccc tca gct ggc ccc aca ggt ccc 1488
Pro Thr Ala Gly Pro Thr Gly Pro Pro Ser Ala Gly Pro Thr Gly Pro
375 380 385 390
ccc act gct ggc cct tct acg gcc act act gtg cct ttg agt ccg gtg 1536
Pro Thr Ala Gly Pro Ser Thr Ala Thr Thr Val Pro Leu Ser Pro Val
395 400 405
gac gat gcc tgc aac gtg aac atc ttc gac gcc atc gcg gag att ggg 1584
Asp Asp Ala Cys Asn Val Asn Ile Phe Asp Ala Ile Ala Glu Ile Gly
410 415 420
aac cag ctg tat ttg ttc aag gat ggg aag tac tgg cga ttc tct gag 1632
Asn Gln Leu Tyr Leu Phe Lys Asp Gly Lys Tyr Trp Arg Phe Ser Glu
425 430 435
ggc agg ggg agc cgg ccg cag ggc ccc ttc ctt atc gcc gac aag tgg 1680
Gly Arg Gly Ser Arg Pro Gln Gly Pro Phe Leu Ile Ala Asp Lys Trp
440 445 450
ccc gcg ctg ccc cgc aag ctg gac tcg gtc ttt gag gag ccg ctc tcc 1728
Pro Ala Leu Pro Arg Lys Leu Asp Ser Val Phe Glu Glu Pro Leu Ser
455 460 465 470
aag aag ctt ttc ttc ttc tct ggg cgc cag gtg tgg gtg tac aca ggc 1776
Lys Lys Leu Phe Phe Phe Ser Gly Arg Gln Val Trp Val Tyr Thr Gly
475 480 485
gcg tcg gtg ctg ggc ccg agg cgt ctg gac aag ctg ggc ctg gga gcc 1824
Ala Ser Val Leu Gly Pro Arg Arg Leu Asp Lys Leu Gly Leu Gly Ala
490 495 500
gac gtg gcc cag gtg acc ggg gcc ctc cgg agt ggc agg ggg aag atg 1872
Asp Val Ala Gln Val Thr Gly Ala Leu Arg Ser Gly Arg Gly Lys Met
505 510 515
ctg ctg ttc agc ggg cgg cgc ctc tgg agg ttc gac gtg aag gcg cag 1920
Leu Leu Phe Ser Gly Arg Arg Leu Trp Arg Phe Asp Val Lys Ala Gln
520 525 530
atg gtg gat ccc cgg agc gcc agc gag gtg gac cgg atg ttc ccc ggg 1968
Met Val Asp Pro Arg Ser Ala Ser Glu Val Asp Arg Met Phe Pro Gly
535 540 545 550
gtg cct ttg gac acg cac gac gtc ttc cag tac cga gag aaa gcc tat 2016
Val Pro Leu Asp Thr His Asp Val Phe Gln Tyr Arg Glu Lys Ala Tyr
555 560 565
ttc tgc cag gac cgc ttc tac tgg cgc gtg agt tcc cgg agt gag ttg 2064
Phe Cys Gln Asp Arg Phe Tyr Trp Arg Val Ser Ser Arg Ser Glu Leu
570 575 580
aac cag gtg gac caa gtg ggc tac gtg acc tat gac atc ctg cag tgc 2112
Asn Gln Val Asp Gln Val Gly Tyr Val Thr Tyr Asp Ile Leu Gln Cys
585 590 595
cct gag gac 2121
Pro Glu Asp
600

<210> 4
<211> 707
<212> PRT
<213> Homo sapiens

<400> 4
Met Ser Leu Trp Gln Pro Leu Val Leu Val Leu Leu Val Leu Gly Cys
-105 -100 -95
Cys Phe Ala Ala Pro Arg Gln Arg Gln Ser Thr Leu Val Leu Phe Pro
-90 -85 -80 -75
Gly Asp Leu Arg Thr Asn Leu Thr Asp Arg Gln Leu Ala Glu Glu Tyr
-70 -65 -60
Leu Tyr Arg Tyr Gly Tyr Thr Arg Val Ala Glu Met Arg Gly Glu Ser
-55 -50 -45
Lys Ser Leu Gly Pro Ala Leu Leu Leu Leu Gln Lys Gln Leu Ser Leu
-40 -35 -30
Pro Glu Thr Gly Glu Leu Asp Ser Ala Thr Leu Lys Ala Met Arg Thr
-25 -20 -15
Pro Arg Cys Gly Val Pro Asp Leu Gly Arg Phe Gln Thr Phe Glu Gly
-10 -5 -1 1 5
Asp Leu Lys Trp His His His Asn Ile Thr Tyr Trp Ile Gln Asn Tyr
10 15 20
Ser Glu Asp Leu Pro Arg Ala Val Ile Asp Asp Ala Phe Ala Arg Ala
25 30 35

Phe Ala Leu Trp Ser Ala Val Thr Pro Leu Thr Phe Thr Arg Val Tyr
    40                  45                  50
Ser Arg Asp Ala Asp Ile Val Ile Gln Phe Gly Val Ala Glu His Gly
55                  60                  65                  70
Asp Gly Tyr Pro Phe Asp Gly Lys Asp Gly Leu Leu Ala His Ala Phe
                75                  80                  85
Pro Pro Gly Pro Gly Ile Gln Gly Asp Ala His Phe Asp Asp Asp Glu
            90                  95                  100
Leu Trp Ser Leu Gly Lys Gly Val Val Val Pro Thr Arg Phe Gly Asn
        105                 110                 115
Ala Asp Gly Ala Ala Cys His Phe Pro Phe Ile Phe Glu Gly Arg Ser
    120                 125                 130
Tyr Ser Ala Cys Thr Thr Asp Gly Arg Ser Asp Gly Leu Pro Trp Cys
135                 140                 145                 150
Ser Thr Thr Ala Asn Tyr Asp Thr Asp Asp Arg Phe Gly Phe Cys Pro
                155                 160                 165
Ser Glu Arg Leu Tyr Thr Arg Asp Gly Asn Ala Asp Gly Lys Pro Cys
            170                 175                 180
Gln Phe Pro Phe Ile Phe Gln Gly Gln Ser Tyr Ser Ala Cys Thr Thr
        185                 190                 195
Asp Gly Arg Ser Asp Gly Tyr Arg Trp Cys Ala Thr Thr Ala Asn Tyr
    200                 205                 210
Asp Arg Asp Lys Leu Phe Gly Phe Cys Pro Thr Arg Ala Asp Ser Thr
215                 220                 225                 230
Val Met Gly Gly Asn Ser Ala Gly Glu Leu Cys Val Phe Pro Phe Thr
                235                 240                 245
Phe Leu Gly Lys Glu Tyr Ser Thr Cys Thr Ser Glu Gly Arg Gly Asp
            250                 255                 260
Gly Arg Leu Trp Cys Ala Thr Thr Ser Asn Phe Asp Ser Asp Lys Lys
        265                 270                 275
Trp Gly Phe Cys Pro Asp Gln Gly Tyr Ser Leu Phe Leu Val Ala Ala
    280                 285                 290
His Glu Phe Gly His Ala Leu Gly Leu Asp His Ser Ser Val Pro Glu
295                 300                 305                 310
Ala Leu Met Tyr Pro Met Tyr Arg Phe Thr Glu Gly Pro Pro Leu His
                315                 320                 325
Lys Asp Asp Val Asn Gly Ile Arg His Leu Tyr Gly Pro Arg Pro Glu
            330                 335                 340
Pro Glu Pro Arg Pro Pro Thr Thr Thr Thr Pro Gln Pro Thr Ala Pro
        345                 350                 355
Pro Thr Val Cys Pro Thr Gly Pro Pro Thr Val His Pro Ser Glu Arg
    360                 365                 370
Pro Thr Ala Gly Pro Thr Gly Pro Pro Ser Ala Gly Pro Thr Gly Pro
375                 380                 385                 390
Pro Thr Ala Gly Pro Ser Thr Ala Thr Thr Val Pro Leu Ser Pro Val
                395                 400                 405
Asp Asp Ala Cys Asn Val Asn Ile Phe Asp Ala Ile Ala Glu Ile Gly
            410                 415                 420
Asn Gln Leu Tyr Leu Phe Lys Asp Gly Lys Tyr Trp Arg Phe Ser Glu
        425                 430                 435
Gly Arg Gly Ser Arg Pro Gln Gly Pro Phe Leu Ile Ala Asp Lys Trp
    440                 445                 450
Pro Ala Leu Pro Arg Lys Leu Asp Ser Val Phe Glu Glu Pro Leu Ser
455                 460                 465                 470
Lys Lys Leu Phe Phe Phe Ser Gly Arg Gln Val Trp Val Tyr Thr Gly
                475                 480                 485
Ala Ser Val Leu Gly Pro Arg Arg Leu Asp Lys Leu Gly Leu Gly Ala
            490                 495                 500
Asp Val Ala Gln Val Thr Gly Ala Leu Arg Ser Gly Arg Gly Lys Met
        505                 510                 515
Leu Leu Phe Ser Gly Arg Arg Leu Trp Arg Phe Asp Val Lys Ala Gln
    520                 525                 530
Met Val Asp Pro Arg Ser Ala Ser Glu Val Asp Arg Met Phe Pro Gly
535                 540                 545                 550
Val Pro Leu Asp Thr His Asp Val Phe Gln Tyr Arg Glu Lys Ala Tyr
                555                 560                 565
Phe Cys Gln Asp Arg Phe Tyr Trp Arg Val Ser Ser Arg Ser Glu Leu
            570                 575                 580
Asn Gln Val Asp Gln Val Gly Tyr Val Thr Tyr Asp Ile Leu Gln Cys
        585                 590                 595
Pro Glu Asp
    600

<210>  5
<211>  32
<212>  DNA
<213>  Artificial Sequence

<220>
<223>  Oligonucleotide designed to act as primer for amplifying DNA
       encoding chsp60 derived from Chlamydia pneumoniae strain KKpn-1.

<400>  5
ggaaggcata tggcagcgaa aaatattaaa ta                                    32

<210>  6
<211>  34
<212>  DNA
<213>  Artificial Sequence

<220>
<223>  Oligonucleotide designed to act as primer for amplifying DNA
       encoding chsp60 derived from Chlamydia pneumoniae strain KKpn-1.

<400>  6
ggaaggcata tgctagtagt ccattcctgc gctt                                  34

## Description

### Technical Field

The present invention relates to a method and kit for screening for a therapeutic drug for arteriosclerosis and a prophylactic/therapeutic drug for complications thereof. More specifically, the present invention relates to a screening method for a therapeutic drug for arteriosclerosis and a prophylactic/therapeutic drug for complications thereof, which comprises using a 60-kDa heat shock protein (hereinafter sometimes abbreviated as "HSP60") derived from *Chlamydia pneumoniae* (hereinafter sometimes abbreviated as "Cp") and cells of an established monocytic cell line of a mammal, and a kit therefor.

### Background Art

*Chlamydia pneumoniae*, an obligate intracellular parasitic Gram-negative bacterium, is a major causal bacterium for community-acquired pneumonia, and has been suggested to be associated with chronic diseases such as arteriosclerosis, asthma, chronic obstructive pulmonary disease, Alzheimer's disease, and multiple sclerosis.

Arteriosclerosis (particularly atherosclerosis) causes complications ranking high as causes of death in Japan, the USA, and Europe, such as acute coronary syndrome (ACS, including acute myocardial infarction and unstable angina pectoris) and cerebrovascular diseases (particularly ischemic cerebrovascular disorders, including cerebral infarction, cerebral embolism, cerebral thrombosis, and the like). The relationship between Cp and arteriosclerosis has been drawing attention since a survey on traffic accident victims reported a correlation between arteriosclerotic lesions and serum anti-Cp antibody prevalence (Saikku, P. et al., Lancet (UK), 1988, Vol. 2, pp. 983-986). Later, much evidence based on seroepidemiological findings has been reported for Cp as a causal microorganism in arteriosclerosis (see, for example, Thomas, M. et al., Circulation (USA), 1999, Vol. 99, pp. 2733-2736). Furthermore, in experimental animal models, Cp infection was reported to promote the formation of arteriosclerotic lesions, and it was also shown that the lesion formation is suppressed by antibacterial drug administration (see, for example, Muhlestein, J.B. et al., Circulation (USA), 1998, Vol. 97, pp. 633-636).

Based on these findings, a clinical study with an antibacterial macrolide was conducted in about 200 patients with myocardial infarction, and recurrence rate of the heart attack was reported to decrease to less than one third. However, in a large-scale long-term study of the same drug in about 7000 subjects conducted with this result in mind in the USA, the drug was determined to be ineffective; the disputes on the efficacy of antibacterial drug remain unsettled. Furthermore, problems including the emergence of resistant strains due to overuse of antibacterial drugs, have been pointed out; even if their efficacy is confirmed, it is postulated that antibacterial drugs will never become the mainstream of the treatment for arteriosclerosis.

The molecular mechanisms behind the formation and progression of arteriosclerotic lesions due to Cp infection have not well been understood to date. Recently, Cp-derived HSP60 (hereinafter sometimes abbreviated as "chsp60") was detected in human arteriosclerotic lesions (Kol, A. et al., Circulation (USA), 1998, Vol. 98, pp. 300-307), and it was reported that when primary macrophages differentiated from human monocytes were stimulated with chsp60, the production of matrix metalloproteinase-9 (hereinafter sometimes abbreviated as "MMP-9"), one of collagenases, was increased (Vehmaan-Kreula, P. et al., Arterioscler. Thromb. Vasc. Biol. (USA), 2001, Vol. 21, pp. e1-e8). MMP-9 is overproduced in progressed human arteriosclerotic lesions, and highly expressed and activated particularly in ruptured lesions (Loftus, A. et al., Stroke (USA), 2000, Vol. 31, pp. 40-47). It has also been shown that high MMP-9 concentrations in plasma can be a risk factor of cardiovascular disease death, that polymorphism in the MMP-9 gene is significantly correlated with the future incidence rate of cardiovascular events in patients with stable angina pectoris (Blankenberg, S. et al., Circulation (USA), 2003, Vol. 107, pp. 1579-1585), and that in MMP-9 knockout mice, the progression of lesions in atherosclerosis is suppressed (Luttun, A. et al., Circulation (USA), 2004, Vol. 109, pp. 1408-1414).

Although atherosclerosis refers to a condition characterized by vascular luminal stenosis by atheroma (plaque) covered by collagen fiber, it is considered, from clinical findings, that plaque properties, rather than degree of stenosis, are important in coronary artery events with thrombus formation that leads to the onset of ACS, such as myocardial infarction and angina pectoris. That is, the hypothesis that lesions with a large lipid core and a thin fibrous cap covering the core are likely to undergo plaque rupture and are at a high risk of developing complications, has been supported (Libby, P., Circulation (USA), 1995, Vol. 91, pp. 2844-2850).

The primary component of the fibrous cap covering the plaque surface is collagen, and matrix metalloproteases (MMPs), a group of collagen-degradative enzymes, are capable of directly weakening the cap (Young, J.L. et al., Thrombosis and Haemostasis (Germany), 2002, Vol. 88, pp. 554-567; Jones, C.B. et al., Cardiovascular Research (Netherlands), 2003, Vol. 59, pp. 812-823). Plaque rupture is considered to occur as macrophages and vascular smooth muscle cells activated by T cells incorporate oxidized LDL to become foamy and the fibrous cap is weakened by MMPs secreted from the resulting foam cells. In particular, MMP-9 is considered to play a major role in plaque destabilization because significantly increased production thereof has been confirmed in ruptured plaques.

Judging from the findings above, as mechanisms behind arteriosclerosis progression due to Cp infection, it is strongly suggested that MMP-9 secretion from macrophages and the like may be promoted by chsp60 stimulation, which MMP-9 may weaken the fibrous cap and cause plaque rupture.

In fact, there have been many reports that drugs exhibiting suppressive action on MMP-9 secretion and production have an effect to suppress the progression of arteriosclerosis. For example, lacidipine, which is a therapeutic drug for hypertension, suppressed MMP-9 secretion and production in mouse macrophages stimulated with phorbol ester or tumor necrosis factor (TNF-α) (Bellosta, S. et al., The Journal of Pharmacology and Experimental Therapeutics (USA), 2001, Vol. 296, No.3, pp. 736-743), and results of a clinical study demonstrated that lacidipine has a suppressive effect on the progression of arteriosclerosis (Zanchetti, A. et al., Circulation (USA), 2002, Vol. 106, pp. 2422-2427). Also, fluvastatin and simvastatin, which are known as cholesterol-reducing drugs, inhibited MMP-9 secretion and production in mouse and human macrophages stimulated with phorbol ester (TPA) (Bellosta, S. et al., Arteriosclerosis, Thrombosis, and Vascular Biology (USA), 1998, Vol. 18, pp. 1671-1678), many statin-series drugs, including these, were shown to have a suppressive effect on the progression of arteriosclerosis in a plurality of clinical studies (Grobbee, D.E. et al., Drugs (New Zealand), 2003, Vol. 63, pp. 893-911).

### Disclosure of Invention

Judging from the above-described findings, a substance capable of suppressing the overproduction of MMP-9 from macrophages and the like due to Cp infection or chsp60 stimulation is expected to become a novel drug effective in the treatment and suppression of progression of arteriosclerosis (particularly prevention of serious complications such as ACS and ischemic cerebrovascular disorders) or the treatment of complications thereof. However, no compound screening system that possesses a sufficient sensitivity to permit use as a search and evaluation system for such drugs, and that is excellent in reproducibility, has been known to date. As with the above-described pharmacological evaluation systems for statin-series drugs and lacidipine, it is possible to induce high levels of MMP-9 production by stimulation with phorbol ester or TNF-α, but induction of MMP-9 production with these drugs or cytokines does not reflect actual Cp infection events, and there have been drawbacks, including conventionally used monocytes and macrophages produce high background values without stimulation due to constitutive expression of MMP-9.

Accordingly, it is an object of the present invention to provide a screening system capable of selecting a substance capable of suppressing the overproduction of MMP-9 from macrophages and the like due to Cp infection or chsp60 stimulation, with high sensitivity, high reproducibility, and high efficiency, and hence to provide a novel therapeutic drug for arteriosclerosis and a prophylactic/therapeutic drug for complications thereof.

With the aim of accomplishing the above-described objectives, the present inventors conducted diligent investigations, and for the first time found that when cells of an established monocytic cell line (e.g., THP-1 cell line) are used as an MMP-9-producing cell and infected with Cp or stimulated with chsp60, remarkably higher MMP-9 expression is unexpectedly induced than with monocytes isolated from blood or with primary macrophages differentiated therefrom. The present inventors conducted further investigations based on these findings, and developed the present invention.

Accordingly, the present invention relates to:
[1] a screening method for a therapeutic substance for arteriosclerosis and/or a prophylactic/therapeutic substance for complications thereof, which comprises using cells of an established monocytic cell line and a *Chlamydia pneumoniae-*derived 60-kDa heat shock protein or a partial peptide thereof or a salt thereof or cells capable of producing the same (the cells may be identical to, or different from, the cells of the established monocytic cell line),
[2] the screening method described in [1] above, which comprises stimulating cells of an established monocytic cell line with a *Chlamydia pneumoniae*-derived 60-kDa heat shock protein or a partial peptide thereof or a salt thereof in the presence and absence of a test compound, and comparing the expressions of the matrix metalloproteinase-9 gene under both conditions,
[3] the screening method described in [1] above, which comprises infecting cells of an established monocytic cell line with *Chlamydia pneumoniae* in the presence and absence of a test compound, and comparing the expressions of the matrix metalloproteinase-9 gene under both conditions,
[4] the screening method described in [1] above, which comprises comparing the expressions of the matrix metalloproteinase-9 gene in cells of an established monocytic cell line capable of producing a *Chlamydia pneumoniae*-derived 60-kDa heat shock protein or a partial peptide thereof or a salt thereof in the presence and absence of a test compound,
[5] the screening method described in [1] above, which comprises using cells of an established monocytic cell line wherein the expression of the matrix metalloproteinase-9 gene with 48 hours of stimulation with 100nM of a *Chlamydia pneumoniae*-derived 60-kDa heat shock protein or a partial peptide thereof or a salt thereof is not less than 5 times the expression without the stimulation,
[6] the screening method described in [1] above, wherein the cells of the established monocytic cell line show substantially no expression of the matrix metalloproteinase-9 gene without stimulation,
[7] the screening method described in [1] above, wherein the cells of the established monocytic cell line are cells of an established cell line selected from the group consisting of THP-1, U-937, P31/FUJ, RAW264.7, and J774A.1,
[8] the screening method described in [1] above, wherein the cells of the established monocytic cell line are cells of the established THP-1 cell line,
[9] a screening kit for a therapeutic substance for arteriosclerosis and/or a prophylactic/therapeutic substance for complications thereof, which comprises cells of an established monocytic cell line, and a *Chlamydia pneumoniae-*derived 60-kDa heat shock protein or a partial peptide thereof or a salt thereof or cells capable of producing the same (the cells may be identical to, or different from, the cells of the established monocytic cell line),
[10] the screening kit described in [9] above, wherein the cells capable of producing a *Chlamydia pneumoniae*-derived 60-kDa heat shock protein or a partial peptide thereof or a salt thereof are cells of *Chlamydia pneumoniae* or an established monocytic cell line,
[11] the screening kit described in [9] above, wherein the cells of the established monocytic cell line are cells of an established cell line selected from the group consisting of THP-1, U-937, P31/FUJ, RAW264.7 and J774A.1,
[12] the screening kit described in [9] above, wherein the cells of the established monocytic cell line are cells of the established THP-1 cell line,
[13] a therapeutic agent for arteriosclerosis and/or a prophylactic/therapeutic agent for complications thereof, which comprises a substance capable of suppressing the induction of the expression of the matrix metalloproteinase-9 gene by a *Chlamydia pneumoniae*-derived 60-kDa heat shock protein in mammalian cells,
[14] a therapeutic method for arteriosclerosis and/or a prophylactic or therapeutic method for complications thereof in a mammal, which comprises administering to the mammal an effective amount of a substance capable of suppressing the induction of the expression of the matrix metalloproteinase-9 gene by a *Chlamydia pneumoniae*-derived 60-kDa heat shock protein in mammalian cells, and
[15] a use of a substance capable of suppressing the induction of the expression of the matrix metalloproteinase-9 gene by a *Chlamydia pneumoniae*-derived 60-kDa heat shock protein in mammalian cells, for producing a therapeutic agent for arteriosclerosis and/or a prophylactic or therapeutic agent for complications thereof, and the like.

### Brief Description of the Drawings

Figure 1 shows changes over days in the induction of TNFα (FIG.1A) and MMP-9 (FIG.1B) production by chsp60 stimulation in cells of an established monocytic cell line.
Figure 2 shows the chsp60 concentration dependence of the induction of MMP-9 production in cells of an established monocytic cell line.

### Detailed Description of the Invention

The screening method of the present invention for a therapeutic substance for arteriosclerosis and/or a prophylactic/therapeutic substance for complications thereof comprises using cells of an established monocytic cell line, a *Chlamydia pneumoniae*-derived 60-kDa heat shock protein or a partial peptide thereof or a salt thereof or cells capable of producing the same. As used herein, "therapy (treatment) of arteriosclerosis" means, for example, suppression of progression of arteriosclerotic lesions, plaque stabilization and the like; as examples of "complications of arteriosclerosis", cardiovascular and cerebrovascular events, including acute coronary syndrome (ACS) such as acute myocardial infarction and unstable angina pectoris, and ischemic cerebrovascular disorders such as cerebral infarction, cerebral thrombosis, and cerebral embolism, can be mentioned.

The chsp60 used in the present invention is not subject to limitation as to the derivation thereof, as long as it is a protein comprising the same or substantially the same amino acid sequence as the amino acid sequence shown by SEQ ID NO:2; for example, the chsp60 can be isolated from various strains belonging to *Chlamydia pneumoniae* separated from nature (for example, clinical isolates from pneumonia patients and the like). The chsp60 may also be a protein chemically synthesized on the basis of the amino acid sequence shown by SEQ ID NO:2, or may also a recombinant protein produced by a transformant incorporating a nucleic acid having the base sequence encoding the above-described amino acid sequence. Alternatively, the chsp60 may also be a protein biochemically synthesized using a cell-free (transcription) translation system with the nucleic acid as a template.

As "substantially the same amino acid sequence as the amino acid sequence shown by SEQ ID NO:2", an amino acid sequence having a homology of about 60% or more, preferably about 70% or more, more preferably about 80% or more, particularly preferably about 90% or more, and most preferably about 95% or more, to the amino acid sequence shown by SEQ ID NO:2, and the like can be mentioned.

As used herein, "homology" means the proportion (%) of the same and similar amino acid residues to all overlapping amino acid residues in the optimal alignment where two amino acid sequences are aligned using a mathematical algorithm known in the relevant technical field (preferably, the algorithm is such that a gap can be introduced into one or both of the sequences for the optimal alignment). A "similar amino acid" means an amino acid having similar physiochemical properties; for example, amino acids classified into the same group, such as aromatic amino acids (Phe, Trp, Tyr), aliphatic amino acids (Ala, Leu, Ile, Val), polar amino acids (GIn, Asn), basic amino acids (Lys, Arg, His), acidic amino acids (Glu, Asp), amino acids having a hydroxyl group (Ser, Thr), and amino acids having a small side chain (Gly, Ala, Ser, Thr, Met), can be mentioned. Substitution by such a similar amino acid is expected to produce no change in the phenotype of protein (i.e., conservative amino acid substitution). Specific examples of conservative amino acid substitution are well known in the relevant technical field and described in various pieces of the literature (see, for example, Bowie et al., Science, 247: 1306-1310 (1990)).

Algorithms to determine the homology of an amino acid sequence include, for example, but are not limited to, the algorithm described in Karlin et al., Proc. Natl. Acad. Sci. USA, 90: 5873-5877 (1993) [the algorithm is incorporated in the NBLAST and XBLAST programs (version 2.0) (Altschul et al., Nucleic Acids Res., 25: 3389-3402 (1997))], the algorithm described in Needleman et al., J. Mol. Biol., 48: 444-453 (1970) [the algorithm is incorporated in the GAP program in the GCG software package], the algorithm described in Myers and Miller, CABIOS, 4: 11-17 (1988) [the algorithm is incorporated in the ALIGN program (version 2.0), which is part of the CGC sequence alignment software package], the algorithm described in Pearson et al., Proc. Natl. Acad. Sci. USA, 85: 2444-2448 (1988) [the algorithm is incorporated in the FASTA program in the GCG software package] and the like.

More preferably, substantially the same amino acid sequence as the amino acid sequence shown by SEQ ID NO:2 is an amino acid sequence having an identity of about 60% or more, preferably about 70% or more, more preferably about 80% or more, and particularly preferably about 90% or more, to the amino acid sequence shown by SEQ ID NO:2.

"A protein comprising substantially the same amino acid sequence as the amino acid sequence shown by SEQ ID NO:2" refers to a protein comprising the aforementioned "substantially the same amino acid sequence as the amino acid sequence shown by SEQ ID NO:2", and having substantially the same quality of activity as a protein comprising the amino acid sequence shown by SEQ ID NO:2.

As used herein, "activity" refers to MMP-9-expression-enhancing activity or plaque destabilization (fibrous coat weakening) activity in cells (for example, cells present in blood or vascular walls, such as leukocytes, including monocytic cells such as monocytes and macrophages, and vascular smooth muscle cells) of mammals (e.g., humans, monkeys, cattle, horses, swine, sheep, goat, dogs, cats, rabbits, hamsters, guinea pigs, mice, rats and the like); "substantially the same quality" means that the activities are qualitatively (e.g., physiologically or pharmacologically) equivalent to each other. It is preferable, therefore, that MMP-9-expression-enhancing activity and the like be equivalent to each other (e.g., 0.5 to 2 times), but quantitative factors such as the extent of these activities and the molecular weights of the proteins may be different.

A measurement of MMP-9-expression-enhancing activity can be performed according to, for example, the methods described in the 5th non-patent document above and the like (ELISA using anti-MMP-9 antibody, gelatin zymography, quantitative RT-PCR) or a method based thereon.

Examples of the chsp60 used in the present invention also include what are called muteins such as proteins comprising (1) an amino acid sequence having one or two or more amino acids (for example, about 1 to 50, preferably about 1 to 30, more preferably about 1 to 10, and particularly preferably several (1 to 5) amino acids) deleted from the amino acid sequence shown by SEQ ID NO:2, (2) an amino acid sequence having one or two or more amino acids (for example, about 1 to 50, preferably about 1 to 30, more preferably about 1 to 10, and particularly preferably several (1 to 5) amino acids) added to the amino acid sequence shown by SEQ ID NO:2, (3) an amino acid sequence having one or two or more amino acids (for example, about 1 to 50, preferably about 1 to 30, more preferably about 1 to 10, and particularly preferably several (1 to 5) amino acids) inserted in the amino acid sequence shown by SEQ ID NO:2, (4) an amino acid sequence having one or two or more amino acids (for example, about 1 to 50, preferably about 1 to 30, more preferably about 1 to 10, and particularly preferably several (1 to 5) amino acids) substituted by other amino acids in the amino acid sequence shown by SEQ ID NO:2, or (5) an amino acid sequence comprising a combination thereof.

When an amino acid sequence is inserted, deleted or substituted as described above, the position of the insertion, deletion or substitution is not subject to limitation.

The chsp60 in the present invention is preferably a protein having the amino acid sequence shown by SEQ ID NO:2, and derived from the *Chlamydia pneumoniae* KKpn-1 strain [Kishimoto T. and Soejima R., Intern. Med., 32: 934-937 (1993)] [the recombinant Escherichia coli BL21(DE3)/pHSP21-1 strain incorporating the chsp60 gene derived from the KKpn-1 strain has been deposited with the International Patent Organism Depositary, National Institute of Advanced Industrial Science and Technology (Central 6, 1-1-1, Higashi, Tsukuba, Ibaraki, 305-8566 Japan) under the accession number of FERM BP-8430 since July 16, 2003], or a variant thereof derived from another Cp strain.

In the present description, proteins and peptides are denoted with the N-terminus (amino terminus) described as the left end and the C-terminus (carboxyl terminus) as the right end, in accordance with the common way of describing peptides. The chsp60, including a protein comprising the amino acid sequence shown by SEQ ID NO:2, may have any of a carboxyl group (-COOH), a carboxylate (-COO⁻), an amide (-CONH₂) or an ester (-COOR) at the C-terminus thereof.

As used herein, R in the ester is exemplified by C₁₋₆ alkyl groups, for example, methyl, ethyl, n-propyl, isopropyl, n-butyl and the like; C₃₋₈ cycloalkyl groups, for example, cyclopentyl, cyclohexyl and the like; C₆₋₁₂ aryl groups, for example, phenyl, α-naphthyl and the like; phenyl-C₁₋₂ alkyl groups, for example, benzyl, phenethyl and the like; C₇₋₁₄ aralkyl groups such as α,-naphthyl-C₁₋₂-alkyl groups such as α-naphthylmethyl; pivaloyloxymethyl groups; and the like.

When the chsp60 has a carboxyl group (or carboxylate) at a position other than the C-terminus, the carboxyl group may be amidated or esterified, and such proteins are also included in the chsp60. As examples of the ester used in this case, the above-described esters at the C-terminus can be mentioned.

Furthermore, the chsp60 also includes those wherein the amino group of the amino acid residue at the N-terminus (e.g., methionine residue) is protected with a protecting group (for example, C₁₋₆ acyl groups such as C₁₋₆ alkanoyls such as formyl group and acetyl group); those wherein the glutamine residue at the N-terminus, which is produced upon cleavage in vivo, is pyroglutaminated; those wherein a substituent (for example, - OH, -SH, amino group, imidazole group, indole group, guanidino group and the like) on the side chain of an amino acid in the molecule is protected with an appropriate protecting group (for example, C₁₋₆ acyl groups such as C₁₋₆ alkanoyl groups such as formyl group and acetyl group, and the like); conjugated proteins such as what are called glycoproteins having sugar chains bound thereto, and the like.

A partial peptide of the chsp60 (hereinafter sometimes simply abbreviated as "the partial peptide of the present invention") may be any peptide having a partial amino acid sequence of the above-described chsp60, and having substantially the same quality of activity as that of the chsp60. As used herein, "substantially the same quality of activity" has the same meaning as described above. A measurement of "substantially the same quality of activity" can be performed in the same manner as the chsp60.

Specifically, examples of the partial peptide of the present invention include one having a partial amino acid sequence comprising a region involved in the MMP-9-expression-enhancing action in mammalian cells, in the amino acid sequence shown by SEQ ID NO:2.

The partial peptide of the present invention is preferably a peptide having at least 50 or more, preferably 100 or more, and more preferably 200 or more amino acids.

Also, the partial peptide of the present invention may have any of a carboxyl group (-COOH), a carboxylate (-COO⁻), an amide (-CONH₂), or an ester (-COOR) at the C-terminus thereof. As used herein, R in the ester is exemplified by the same examples as those mentioned with respect to the chsp60. When the partial peptide of the present invention has a carboxyl group (or carboxylate) at a position other than the C-terminus, the carboxyl group may be amidated or esterified, and such partial peptides are also included in the partial peptide of the present invention. As examples of the ester used in this case, the above-described esters at the C-terminus can be mentioned.

Furthermore, the partial peptide of the present invention, like the above-described chsp60, also includes those wherein the amino group of the amino acid residue at the N-terminus is protected with a protecting group; those wherein the glutamine residue at the N-terminus is pyroglutaminated; those wherein a substituent on the side chain of an amino acid in the molecule is protected with an appropriate protecting group; conjugated peptides such as what are called glycopeptides having sugar chains bound thereto, and the like.

As the salt of the chsp60 or a partial peptide thereof, physiologically acceptable salts with acids (e.g., inorganic acids, organic acids), bases (e.g., alkali metal salts) and the like can be mentioned, with preference given to physiologically acceptable acid adduct salts. Examples of such salts include salts with inorganic acids (for example, hydrochloric acid, phosphoric acid, hydrobromic acid, sulfuric acid), salts with organic acids (for example, acetic acid, formic acid, propionic acid, fumaric acid, maleic acid, succinic acid, tartaric acid, citric acid, malic acid, oxalic acid, benzoic acid, methanesulfonic acid, benzenesulfonic acid), and the like.

The chsp60 or a salt thereof can be produced from cells of various Cp strains by protein separation and purification techniques known per se, as described above. Specifically, for example, appropriate host cells such as human lung-derived cultured cells (e.g., HL cells and the like) are infected with Cp, and cultured in a medium suitable for *chlamydia* growth (e.g., Eagle medium supplemented with cycloheximide, and the like) for an appropriate period, and thereafter disrupted by sonication and the like, and Cp cells are mass-purified using density gradient centrifugation and the like. The chsp60 or a salt thereof can be purified by dissolving the Cp cells obtained, extracting a soluble fraction, and then separating the soluble fraction by reverse phase chromatography, ion exchange chromatography, affinity chromatography and the like.

The chsp60 or a partial peptide thereof or a salt thereof (hereinafter sometimes generically referred to as "chsp60 species (chsp60s)") can also be produced according to a known method of peptide synthesis.

The method of peptide synthesis may, for example, be any of solid phase synthesis and liquid phase synthesis. The desired protein can be produced by condensing a partial peptide or amino acids that can constitute a chsp60 species and the remaining portion, and, when the product has a protecting group, removing the protecting group.

Here, condensation and protecting group removal can be achieved by methods known per se, for example, the methods described in (1) to (5) below.
(1) M. Bodanszky and M.A. Ondetti: Peptide Synthesis, Interscience Publishers, New York (1966)
(2) Schroeder and Luebke: The Peptide, Academic Press, New York (1965)
(3) Nobuo Izumiya et al.: Peptide Gosei-no-Kiso to Jikken (Basics and experiments of peptide synthesis), Maruzen Co. (1975)
(4) Haruaki Yajima and Shunpei Sakakibara: Seikagaku Jikken Koza (Biochemical Experiment) 1, Tanpakushitsu no Kagaku (Chemistry of Proteins) IV, 205 (1977)
(5) Haruaki Yajima ed.: Zoku Iyakuhin no Kaihatsu (A Sequel to Development of Pharmaceuticals), Vol. 14, Peptide Synthesis, Hirokawa Shoten

The protein (peptide) thus obtained can be purified and isolated using a known method of purification. Here, as examples of the method of purification, solvent extraction, distillation, column chromatography, liquid chromatography, recrystallization, combinations thereof and the like can be mentioned.

When the protein (peptide) obtained by the above-described method is the free form, the free form can be converted into an appropriate salt by a known method or a method based thereon; conversely, when the protein (peptide) is obtained in the form of a salt, the salt can be converted into the free form or another salt by a known method or a method based thereon.

To synthesize a chsp60 species, commercially available resins for protein synthesis may usually be used. As examples of such resins, chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl)phenoxy resin and the like can be mentioned.

Using such a resin, amino acids having α-amino groups and side-chain functional groups protected as appropriate are condensed on the resin in accordance with the sequence of the desired protein (peptide) according to various methods of condensation known per se. At the end of the reaction, the protein and the like are excised from the resin and the various protecting groups are removed simultaneously; then, an intramolecular disulfide bond-forming reaction is performed in a highly diluted solution to give the desired protein (peptide) or an amide thereof.

For the above-described condensation of protected amino acids, various activation reagents for protein synthesis may be used, with preference given to carbodiimides. Useful carbodiimides include DCC, N,N'-diisopropylcarbodiimide, N-ethyl-N'-(3-dimethylaminopropyl)carbodiimide and the like.

Condensation of protected amino acids is performed by, for example, adding the protected amino acids, along with an activation reagent and a racemization inhibitor (for example, HOBt, HOOBt), directly to the resin, or by adding the protected amino acids to the resin after being previously activated in the form of a symmetric acid anhydride, HOBt ester, or HOOBt ester.

Solvents used in the activation of protected amino acids or condensation with the resin can be selected as appropriate from among solvents known to be usable for protein condensation reactions. For example, acid amides such as N,N-dimethylformamide, N,N-dimethylacetamide, and N-methylpyrrolidone; halogenated hydrocarbons such as methylene chloride and chloroform; alcohols such as trifluoroethanol; sulfoxides such as dimethylsulfoxide; amines such as pyridine; ethers such as dioxane and tetrahydrofuran; nitriles such as acetonitrile and propionitrile; esters such as methyl acetate and ethyl acetate; appropriate mixtures of these solvents, and the like can be used.

Reaction temperature is selected as appropriate from the range known to be useful for protein condensation reactions, and is normally selected as appropriate from the range of about -20°C to 50°C. The activated amino acid derivative is normally used in an excess of 1.5 to 4 times. If a test using the ninhydrin reaction reveals insufficient condensation, the condensation can be completed by repeating the condensation reaction without splitting off the protecting groups. If the condensation is yet insufficient even after repeating the reaction, unreacted amino acids can be acetylated with acetic anhydride or acetylimidazole to ensure sufficient condensation.

Protection methods and protecting groups for the functional groups that should not be involved in the reaction of the starting material amino acid, methods of splitting off the protecting groups, methods of activating the functional groups involved in the reaction, and the like can be selected as appropriate from among known groups or known means.

As examples of the protecting groups for the amino groups of the starting material amino acid, Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc and the like can be mentioned.

A carboxyl group in a starting material can be protected by, for example, alkyl esterification (for example, esterification with a linear, branched or cyclic alkyl such as methyl, ethyl, propyl, butyl, t-butyl, cyclopentyl, cyclohexyl, cycloheptyl, cyclooctyl, or 2-adamantyl), aralkyl esterification (for example, benzyl esterification, 4-nitrobenzyl esterification, 4-methoxybenzyl esterification, 4-chlorobenzyl esterification, benzhydryl esterification), phenacyl esterification, benzyloxycarbonyl hydrazidation, t-butoxycarbonyl hydrazidation, trityl hydrazidation and the like.

The hydroxyl group of serine can be protected by, for example, esterification or etherification. As examples of groups suitable for this esterification, lower (C₁₋₆) alkanoyl groups such as acetyl group, aroyl groups such as benzoyl group, groups derived from carbonic acid, such as benzyloxycarbonyl group and ethoxycarbonyl group, and the like can be mentioned. As examples of groups suitable for the etherification, benzyl group, tetrahydropyranyl group, t-butyl group and the like can be mentioned.

As examples of the protecting group for the phenolic hydroxyl group of tyrosine, Bzl, Cl₂-Bzl, 2-nitrobenzyl, Br-Z, t-butyl and the like can be mentioned.

As examples of the protecting group for the imidazole moiety of histidine, Tos, 4-methoxy-2,3,6-trimethylbenzenesulfonyl, DNP, benzyloxymethyl, Bum, Boc, Trt, Fmoc and the like can be mentioned.

As examples of the method of removing (split off) the protecting group, catalytic reduction in a hydrogen gas stream in the presence of a catalyst such as Pd-black or Pd-carbon; acid treatment with anhydrous hydrogen fluoride, methanesulfonic acid, trifluoromethanesulfonic acid or trifluoroacetic acid, or a mixed solution thereof; treatment with a base such as diisopropylethylamine, triethylamine, piperidine or piperazine; reduction with sodium in liquid ammonia, and the like can be mentioned. The reaction of splitting of the protecting group by the above-described acid treatment is normally performed at a temperature of about -20°C to 40°C. In the acid treatment, addition of a cation scavenger such as anisole, phenol, thioanisole, meta-cresol, para-cresol, dimethylsulfide, 1,4-butanedithiol or 1,2-ethanedithiol is effective. The 2,4-dinitrophenyl group used as the protecting group for the imidazole moiety of histidine is removed by thiophenol treatment. The formyl group used as the protecting group for the indole moiety of tryptophan is removed by alkali treatment with dilute sodium hydroxide solution, dilute ammonia or the like, as well as by the above-described acid treatment in the presence of 1,2-ethanedithiol, 1,4-butanedithiol or the like.

As examples of the activated carboxyl group in a starting material amino acid, corresponding acid anhydrides, azides, activated esters [esters with alcohols (for example, pentachlorophenol, 2,4,5-trichlorophenol, 2,4-dinitrophenol, cyanomethyl alcohol, para-nitrophenol, HONB, N-hydroxysuccimide, N-hydroxyphthalimide, HOBt)], and the like can be mentioned. As examples of the activated amino group in a starting material amino acid, corresponding phosphoric amides can be mentioned.

In another method of obtaining an amide of the protein (peptide), for example, the α-carboxyl group of the carboxy-terminal amino acid is first protected by amidation, and the peptide chain on the amino group side is then extended to a desired length; thereafter, a protein (peptide) having only the protecting group for the N-terminal α-amino group in the peptide chain removed and a protein (peptide) having only the protecting group for the C-terminal carboxyl group removed are prepared, and the two proteins (peptides) are condensed in a mixed solvent as described above. Details of the condensation reaction are the same as those described above. After the protected protein (protected peptide) obtained by the condensation is purified, all the protecting groups can be removed by the above-described method to give the desired crude protein (crude peptide). This crude protein (crude peptide) may be purified by various known means of purification, and the major fraction may be lyophilized to give an amide of the desired protein (peptide).

An ester of the protein (peptide) can be obtained by, for example, condensing the α-carboxyl group of the carboxy-terminal amino acid with a desired alcohol to prepare an amino acid ester, and then following the same procedures as those for the above-described amide of the protein (peptide).

The partial peptide of the present invention or a salt thereof can also be produced by cleaving the chsp60 or a salt thereof with an appropriate peptidase.

Furthermore, a chsp60 species can also be produced by culturing a transformant incorporating an expression vector comprising a nucleic acid encoding the chsp60 or a partial peptide thereof, and separating and purifying the chsp60 species from the culture obtained.

The nucleic acid encoding the chsp60 or a partial peptide thereof may be a DNA or RNA, or may be a DNA/RNA chimera. The nucleic acid is preferably a DNA. Also, the nucleic acid may be double stranded or single stranded. When the nucleic acid is double stranded, it may be a double-stranded DNA, a double-stranded RNA or a DNA:RNA hybrid. When the nucleic acid is single stranded, it may be a sense strand (i.e., coding strand) or an antisense strand (i.e., non-coding strand).

As the DNA encoding the chsp60 or a partial peptide thereof, a genomic DNA extracted from Cp cells by a conventional method, a cDNA prepared by a reverse transcription reaction (or amplified by RT-PCR) from RNA extracted from Cp cells by a conventional method, a chemically synthesized DNA, and the like can be mentioned.

Specifically, as examples of the DNA encoding the chsp60 or a partial peptide thereof, a DNA comprising the base sequence shown by SEQ ID NO:1, or a DNA comprising a base sequence that hybridizes with the base sequence shown by SEQ ID NO:1 under highly stringent conditions, and encoding a protein (peptide) having substantially the same quality of activity (e.g., MMP-9-expression-enhancing activity and the like) as the aforementioned protein comprising the amino acid sequence shown by SEQ ID NO:2, and the like can be mentioned.

As examples of the DNA capable of hybridizing with the base sequence shown by SEQ ID NO:1 under highly stringent conditions, a DNA comprising a base sequence having a homology of about 60% or more, preferably about 70% or more, more preferably about 80% or more, particularly preferably about 90% or more, and most preferably about 95% or more, to the base sequence shown by SEQ ID NO:1 in the overlapping region, and the like can be used.

The hybridization can be performed by a method known per se or a method based thereon, for example, the method described in Molecular Cloning, 2nd edition (J. Sambrook et al., Cold Spring Harbor Lab. Press, 1989) and the like. When a commercially available library is used, the hybridization can be performed according to the method described in the instruction manual attached. The hybridization can be performed preferably under highly stringent conditions.

As used herein, "stringent conditions" refer to conditions wherein the base sequence shown by SEQ ID NO:1 and a DNA having a base sequence having a homology of about 60% or more, preferably about 70% or more, more preferably about 80% or more, particularly preferably about 90% or more, and most preferably about 95% or more, in the overlapping region are hybridizable with each other; for example, conditions of a sodium concentration of about 19 to 40mM, preferably about 19 to 20mM, and a temperature of about 50 to 70°C, preferably about 60 to 65°C, can be mentioned, with preference given to the case of a sodium concentration of about 19mM and a temperature of about 65°C. Those skilled in the art are able to easily obtain desired stringency by changing the salt concentration of the hybridization solution, hybridization reaction temperature, probe concentration, probe length, the number of mismatches, hybridization reaction time, the salt concentration of the washing solution, washing temperature, and the like as appropriate.

The DNA encoding the chsp60 or a partial peptide thereof is preferably a DNA comprising the base sequence shown by SEQ ID NO:1 or a portion of the base sequence with a matching reading frame of the codon (that is, in-frame) or the like.

The DNA encoding the chsp60 or a partial peptide thereof can be cloned by amplification by the PCR method using a synthetic DNA primer having a portion of the base sequence encoding the protein or peptide with a genomic DNA or cDNA derived from chsp60-producing cells (e.g., Cp cells) as a template, or by screening a library prepared by incorporating the genomic DNA fragment or cDNA into an appropriate cloning vector (e.g., bacteriophage, plasmid, cosmid, phagemid and the like) by colony or plaque hybridization with a labeled DNA fragment or synthetic DNA encoding a portion or whole of the chsp60 as a probe. The hybridization can be performed according to, for example, the method described in Molecular Cloning, 2nd edition (ibidem) and the like. When a commercially available library is used, the hybridization can be performed according to the method described in the instruction manual attached to the library. When an expression vector is used as a vector for the library, the DNA encoding the chsp60 or a partial peptide thereof can also be cloned by an antibody screening method using an anti-chsp60 antibody. Also, as a method of obtaining a full-length cDNA clone quickly and conveniently, the RACE method can be used. Hence, a full-length cDNA clone can be obtained by synthesizing oligonucleotides homologous to partial base sequences of an sense strand and antisense strand of the double-stranded DNA corresponding to a portion of the chsp60 cDNA, performing 5' and 3' RACE reactions with each oligonucleotide and an appropriate adapter primer as a pair of PCR primers, and joining the amplified fragments by a method such as using restriction endonuclease and ligase.

Alternatively, the DNA encoding the chsp60 or a partial peptide thereof can be chemically synthesized on the basis of the base sequence shown by SEQ ID NO:1 using a commercially available automated DNA/RNA synthesizer. It is also possible to prepare a full-length DNA by synthesizing a fragment of a sense strand and a fragment of an antisense strand in a mutually overlapping length, hybridizing these fragments, and performing a PCR method and the like in combination.

The base sequence of the DNA can be converted by a method known per se such as the ODA-LA PCR method, the Gapped duplex method, or the Kunkel method, or a method based thereon, using a known kit, for example, Mutan^{™}-super Express Km (Takara Shuzo Co., Ltd.), Mutan^{™}-K (Takara Shuzo Co., Ltd.) and the like.

The cloned protein-encoding DNA can be used as is or, if desired, after digestion with a restriction endonuclease or addition of a linker, depending on the purpose of use. The DNA may have ATG as a translation initiation codon at the 5' end thereof, and may have TAA, TGA or TAG as a translation termination codon at the 3' end thereof. These translation initiation and termination codons may be added using an appropriate synthetic DNA adapter.

An expression vector comprising the nucleic acid encoding the chsp60 or a partial peptide thereof can be produced by, for example, excising the desired DNA fragment from the DNA encoding the chsp60, and ligating the DNA fragment to the downstream of a promoter in an appropriate expression vector.

As the expression vector, plasmids derived from *Escherichia coli* (e.g., pBR322, pBR325, pUC12, pUC13); plasmids derived from *Bacillus subtilis* (e.g., pUB110, pTP5, pC194); plasmids derived from yeast (e.g., pSH19, pSH15); bacteriophages such as λ phage; animal viruses such as retrovirus, vaccinia virus, and baculovirus; pA1-11, pXT1, pRc/CMV, pRc/RSV, pcDNAI/Neo, and the like can be used.

The promoter may be any promoter compartible with the host used for gene expression.

When the host is a bacterium of the genus *Escherichia,* the trp promoter, the lac promoter, the recA promoter, the λP_{L} promoter, the lpp promoter, the T7 promoter and the like are preferable.

When the host is a bacterium of the genus *Bacillus,* the SPO1 promoter, the SPO2 promoter, the penP promoter and the like are preferable.

When the host is a yeast, the PHO5 promoter, the PGK promoter, the GAP promoter, the ADH promoter and the like are preferable.

When the host is an insect cell, the polyhedrin promoter, the P10 promoter and the like are preferable.

For example, when the host is an animal cell, the SRα promoter, the SV40 promoter, the LTR promoter, the CMV (cytomegalovirus) promoter, the HSV-TK promoter and the like can be used. In particular, the CMV promoter, the SRα promoter and the like are preferable.

As the expression vector, one optionally comprising an enhancer, a splicing signal, a poly A addition signal, a selection marker, an SV40 replication origin (hereinafter sometimes abbreviated as SV40ori) and the like, in addition to the above-described examples, can be used. As examples of the selection marker, the ampicillin resistance gene (hereinafter sometimes abbreviated as Amp^{r}), the neomycin resistance gene (hereinafter sometimes abbreviated as Neo^{r}, G418 resistance), the tetracycline resistance gene, the hygromycin resistance gene, the dihydrofolate reductase gene (hereinafter sometimes abbreviated as dhfr, methotrexate (MTX) resistance), and the like can be mentioned.

Also, a signal sequence compatible with the host may be added as necessary to the N-terminal side of the chsp60. When the host is a bacterium of the genus *Escherichia,* a PhoA signal sequence, an OmpA signal sequence and the like can be used; when the host is a bacterium of the genus *Bacillus,* an α-amylase signal sequence, a subtilisin signal sequence and the like can be used; when the host is a yeast, an MFα signal sequence, an SUC2 signal sequence and the like can be used; when the host is an animal cell, an insulin signal sequence, an α-interferon signal sequence, an antibody molecule signal sequence and the like can be used.

A transformant comprising the DNA encoding the chsp60 or a partial peptide thereof can be produced by transforming the host with an expression vector comprising the DNA according to a known method.

Here, as the expression vector, those mentioned above can be mentioned.

As examples of the host used, bacteria of the genus *Escherichia,* bacteria of the genus *Bacillus,* yeasts, insect cells, insects, animal cells and the like can be mentioned.

As examples of the bacteria of the genus *Escherichia, Escherichia coli* K12 DH1 [Proceedings of the National Academy of Sciences of the USA (Proc. Natl. Acad. Sci. USA), Vol. 60, 160 (1968)], JM103 [Nucleic Acids Research, Vol. 9, 309 (1981)], JA221 [Journal of Molecular Biology, Vol. 120, 517 (1978)], HB101 [Journal of Molecular Biology, Vol. 41, 459 (1969)], C600 [Genetics, Vol. 39, 440 (1954)] and the like can be used.

As examples of the bacteria of the genus *Bacillus, Bacillus subtilis* MI114 [Gene, Vol. 24, 255 (1983)], 207-21 [Journal of Biochemistry, Vol. 95, 87 (1984)] and the like can be used.

As examples of the yeasts, *Saccharomyces cerevisiae* AH22, AH22R⁻, NA87-11A, DKD-5D, and 20B-12, *Schizosaccharomyces pombe* NCYC1913 and NCYC2036, *Pichia pastoris* KM71 and the like can be used.

As the insect cells, cells of an established cell line derived from the cabbage armyworm *(Spodoptera frugiperda* cells; Sf cells), MG1 cells derived from the *Trichoplusia* ni mid-intestine, High Five™ cells derived from *Trichoplusia ni* eggs, cells derived from *Mamestra brassicae*, cells derived from *Estigmena acrea,* and the like can be used when the virus is AcNPV, for example. When the virus is BmNPV, cells of an established cell line derived from *Bombyx mori (Bombyx mori* N cells; BmN cells) and the like can be used as the insect cells. As examples of the Sf cells, Sf9 cells (ATCC CRL1711), Sf21 cells (both types of cells are described in Vaughn, J.L. et al., In Vivo, 13, 213-217 (1977)) and the like can be used.

As examples of the insects, *Bombyx mori* larvae and the like can be used [Maeda et al., Nature, Vol. 315, 592 (1985)].

As examples of the animal cells, simian cells COS-7 and Vero, Chinese hamster cells CHO (hereinafter abbreviated as CHO cells), Chinese hamster cells CHO lacking the dhfr gene (hereinafter abbreviated as CHO(dhfr⁻) cells), mouse L cells, mouse AtT-20, mouse myeloma cells, rat GH3, human FL cells and the like can be used.

Transformation can be performed according to a known method depending on the kind of the host.

Bacteria of the genus *Escherichia* can be transformed according to, for example, the methods described in the Proceedings of the National Academy of Sciences of the USA (Proc. Natl. Acad. Sci. USA), Vol. 69, 2110 (1972), Gene, Vol. 17, 107 (1982) and the like.

Bacteria of the genus *Bacillus* can be transformed according to, for example, the methods described in Molecular & General Genetics, Vol. 168, 111 (1979) and the like.

Yeasts can be transformed according to, for example, the methods described in Methods in Enzymology, Vol. 194, 182-187 (1991), Proceedings of the National Academy of Sciences of the USA (Proc. Natl. Acad. Sci. USA), Vol. 75, 1929 (1978) and the like.

Insect cells and insects can be transformed according to, for example, the methods described in Bio/Technology, 6, 47-55 (1988) and the like.

Animal cells can be transformed, for example, according to the methods described in Saibo Kogaku (Cell Engineering), extra issue 8, Shin Saibo Kogaku Jikken Protocol (New Cell Engineering Experimental Protocol), 263-267 (1995), published by Shujunsha, and Virology, Vol. 52, 456 (1973).

Cultivation of a transformant can be performed according to a known method depending on the kind of the host.

For example, when culturing a transformant whose host is a bacterium of the genus *Escherichia* or a bacterium of the genus *Bacillus,* the medium used for cultivation is preferably a liquid medium. The medium preferably contains substances required for the growth of the transformant, such as carbon sources, nitrogen sources, and inorganic substances. As examples of the carbon sources, glucose, dextrin, soluble starch, sucrose and the like can be mentioned; as examples of the nitrogen sources, inorganic or organic substances such as ammonium salts, nitrate salts, corn steep liquor, peptone, casein, beef extract, soybean cake, and potato extract can be mentioned; as examples of the inorganic substances, calcium chloride, sodium dihydrogenphosphate, magnesium chloride and the like can be mentioned. In addition, yeast extract, vitamins, growth promoting factors and the like may be added to the medium. The pH of the medium is preferably about 5 to about 8.

The medium for cultivation of a transformant whose host is a bacterium of the genus *Escherichia* is preferably, for example, an M9 medium containing glucose and Casamino acids [Miller, Journal of Experiments in Molecular Genetics, 431-433, Cold Spring Harbor Laboratory, New York, 1972]. Chemicals such as 3β-indolylacrylic acid may be added to the medium as necessary to allow the promoter to function efficiently.

Cultivation of a transformant whose host is a bacterium of the genus *Escherichia* is normally performed at about 15°C to about 43°C for about 3 to about 24 hours. The culture may be aerated or agitated as necessary.

Cultivation of a transformant whose host is a bacterium of the genus *Bacillus* is normally performed at about 30°C to about 40°C for about 6 to about 24 hours. The culture may be aerated or agitated as necessary.

As examples of the medium for culturing a transformant whose host is a yeast, Burkholder's minimal medium [Bostian, K.L. et al., Proceedings of the National Academy of Sciences of the USA (Proc. Natl. Acad. Sci. USA), Vol. 77, 4505 (1980)], an SD medium containing 0.5% Casamino acid [Bitter, G.A. et al., Proceedings of the National Academy of Sciences of the USA (Proc. Natl. Acad. Sci. USA), Vol. 81, 5330 (1984)] and the like can be mentioned. The pH of the medium is preferably about 5 to about 8. Cultivation is normally performed at about 20°C to about 35°C for about 24 to about 72 hours. The culture may be aerated or agitated as necessary.

As examples of the medium for culturing a transformant whose host is an insect cell or an insect, Grace's Insect Medium [Grace, T.C.C., Nature, 195, 788 (1962)] having additives such as 10% inactivated bovine serum added thereto as appropriate, and the like can be used. The pH of the medium is preferably about 6.2 to about 6.4. Cultivation is normally performed at about 27°C for about 3 days to about 5 days. The culture may be aerated or agitated as necessary.

As examples of the medium for culturing a transformant whose host is an animal cell, an MEM medium containing about 5% to about 20% fetal bovine serum [Science, Vol. 122, 501 (1952)], DMEM medium [Virology, Vol. 8, 396 (1959)], RPMI 1640 medium [The Journal of the American Medical Association, Vol. 199, 519 (1967)], 199 medium [Proceeding of the Society for the Biological Medicine, Vol. 73, 1 (1950)] and the like can be used. The pH of the medium is preferably about 6 to about 8. Cultivation is normally performed at about 30°C to about 40°C for about 15 to about 60 hours. The culture may be aerated or agitated as necessary.

As described above, the chsp60 or a partial peptide thereof or a salt thereof can be produced in or outside the cells of a transformant or in the cell membrane.

The chsp60 species can be separated and purified from the culture obtained by culturing the aforementioned transformant according to a method known per se.

For example, when the chsp60 species is extracted from cultured cells, a method that comprises suspending the cells collected from the culture by a known method in an appropriate buffer, disrupting the cells by ultrasonication, lysozyme and/or freeze-thawing and the like, and performing centrifugation and filtration, to yield a crude extract of the soluble protein, and the like can be used as appropriate. The buffer may contain a protein denaturant such as urea or guanidine hydrochloride and a surfactant such as Triton X-100™. When the protein (peptide) is secreted in the culture broth, a known method comprising collecting a culture supernatant from the culture is used.

The protein (peptide) contained in the culture supernatant or extract thus obtained can be separated and purified according to a method known per se. Such methods include methods based on solubility, such as salting-out and solvent precipitation; methods based mainly on differences in molecular weight, such as dialysis, ultrafiltration, gel filtration, and SDS-polyacrylamide gel electrophoresis; methods based on differences in electric charge, such as ion exchange chromatography; methods based on specific affinity, such as affinity chromatography; methods based on differences in hydrophobicity, such as reverse phase high performance liquid chromatography; methods based on differences in isoelectric point, such as isoelectric focusing; and the like. These methods may be combined as appropriate.

To purify a recombinant chsp60 species more quickly and conveniently, it is preferable to use affinity chromatography. As the affinity column, an anti-chsp60 antibody column can be used; when an appropriate tag sequence (e.g., His tag, GST tag, MBP tag and the like) is added to the DNA encoding the chsp60 or a partial peptide thereof, a column with a metal ion chelate, glutathione, or maltose immobilized thereon and the like can be used as appropriate. Monoclonal antibodies or polyclonal antibodies against the chsp60 are commercially available (e.g., those produced by Amersham Company), or can be produced using an chsp60 species as an antigen according to an antibody or antiserum production method known per se (see, for example, below).

When the protein (peptide) thus obtained is in the free form, the free form can be converted into a salt by a method known per se or a method based thereon; when the protein (peptide) is obtained in the form of a salt, the salt can be converted into the free form or another salt by a method known per se or a method based thereon.

The protein (peptide) produced by the transformant can be treated with a suitable protein-modifying enzyme before or after the purification, so as to make an optionally chosen modification or to partially remove a polypeptide. As examples of the protein-modifying enzyme used, trypsin, chymotrypsin, arginyl endopeptidase, protein kinase, glycosidase and the like can be mentioned.

The presence of the chsp60 species thus obtained can be confirmed by an enzyme immunoassay, Western blotting and the like using a specific antibody.

Alternatively, a chsp60 species can be synthesized in vitro using a cell-free protein translation system comprising rabbit reticulocyte lysate, wheat germ lysate, *Escherichia coli* lysate or the like, with an RNA corresponding to the DNA encoding the above-described chsp60 or a partial peptide thereof as a template. Alternatively, a chsp60 species can also be synthesized using a cell-free transcription/translation system further comprising RNA polymerase, with the DNA encoding the chsp60 or a partial peptide thereof as a template. The cell-free protein (transcription) translation system used may be a commercial product, and may be prepared in accordance with a method known per se; specifically, an *Escherichia coli* extract can be prepared in accordance with the method described in Pratt J.M. et al., Transcription and Translation, 179-209, Hames B.D. & Higgins S.J. eds., IRL Press, Oxford (1984) and the like. Commercially available cell lysates include those derived from *Escherichia* coli such as the E. coli S30 extract system (manufactured by Promega Company) and the RTS 500 Rapid Translation System (manufactured by Roche Company), those derived from rabbit reticulocytes such as the Rabbit Reticulocyte Lysate System (manufactured by Promega Company), and those derived from wheat germ such as PROTEIOS™ (manufactured by TOYOBO Company). Of these, a cell-free protein translation system using a wheat germ lysate is suitable. For preparing a wheat germ lysate, a method described in, for example, Johnston F.B. et al., Nature, 179, 160-161 (1957) or Erickson A.H. et al., Meth. Enzymol., 96, 38-50 (1996) can be used.

As a system or apparatus for protein synthesis, a batch method (Pratt, J.M. et al. (1984), ibidem), a continuous cell-free protein synthesis system wherein amino acids, energy sources and the like are continuously supplied to the reaction system (Spirin A.S. et al., Science, 242, 1162-1164 (1988)), the dialysis method (Kikawa et al., 21st Annual Meeting of the Molecular Biology Society of Japan, WID6), or the overlay method (instruction manual of the PROTEIOS^{™} Wheat germ cell-free protein synthesis core kit: manufactured by TOYOBO) and the like can be mentioned. Furthermore, a method wherein template RNA, amino acids, energy sources and the like are supplied to the synthetic reaction system whenever necessary, and synthetic products and decomposition products are discharged whenever necessary (Japanese Patent Unexamined Publication No. 2000-333673) and the like can be used.

Conversion of the protein (peptide) thus obtained to a salt (free form), modification before or after purification and confirmation (detection) of the presence can be performed in the same manner as described above.

In the screening method of the present invention, a chsp60 species can be provided not only as an isolated protein (peptide), but also in the form of cells capable of producing the same. As cells capable of producing a chsp60 species, not only various strains belonging to *Chlamydia pneumoniae* that produce the chsp60 naturally, but also cells manipulated to artificially produce a chsp60 species, for example, a transformant comprising the aforementioned DNA encoding the chsp60 or a partial peptide thereof, can be mentioned. In the latter case, the transformant is capable of producing a sufficient amount of the chsp60 to enhance MMP-9 expression in cells of an established monocytic cell line under conditions suitable for cultivation of the established cell line.

Preferably, the cells capable of producing a chsp60 species are cells of a bacterial strain belonging to *Chlamydia pneumoniae*, particularly preferably the aforementioned KKpn-1 strain.

The cells of an established monocytic cell line used in the present invention are not subject to limitation, as long as they are cells of a cell line established from mammal-derived monocytes or macrophages, and include not only cells of a known monocytic cell line (for example, human monocyte-derived THP-1, U-937, and P31/FUJ, mouse monocyte-derived RAW264.7, J774A.1, and the like), but also cells of a cell line newly established by culturing monocytes or macrophages isolated from a mammal using a known culturing technique.

Cells of an established monocytic cell line show remarkably higher levels of enhancement of MMP-9 expression by chsp60 stimulation than monocytes and macrophages isolated from a mammal such as humans, or primary culture monocytic cells such as primary macrophages differentiated from monocytes using a differentiation inducer such as GM-CSF. Therefore, they are advantageous in that a more highly sensitive screening system can be provided. It is desirably to use cells of an established monocytic cell line wherein the expression of the MMP-9 gene with 48 hours of stimulation with a 100 nM chsp60 species is not less than 5 times, preferably not less than 10 times, and more preferably not less than 20 times, the expression without the stimulation. Although the expression amount of the MMP-9 gene without chsp60 stimulation is not subject to limitation, it is more preferable that the MMP-9 gene be substantially not expressed (that is, below the limit of detection).

Preferably, the cells of the established monocytic cell line are cells of a human monocyte-derived cell line such as THP-1, U-937, or P31/FUJ, particularly preferably cells of the THP-1 cell line.

The cells of the an established monocytic cell line can be subcultured and maintained in a culture broth suitable for each cell line [for example, minimum essential medium (MEM) supplemented with fetal bovine serum, antibiotics (penicillin, streptomycin and the like) as desired, Dulbecco's modified Eagle medium (DMEM), RPMI1640 medium, 199 medium, F12 medium and the like].

The screening method of the present invention comprises measuring and comparing the expressions of the MMP-9 gene in cells of an established monocytic cell line stimulated with a chsp60 species under two conditions: in the presence and absence of a test compound.

A stimulus with a chsp60 species can be selected as appropriate depending on the form of the chsp60 species provided. That is, when the chsp60 species is an isolated protein (peptide), the stimulation can be performed by incubating cells of an established monocytic cell line using a culture broth for cells of an established monocytic cell line (see, for example, above) wherein the chsp60 species has been dissolved to a predetermined concentration. As the chsp60 species concentration, about 100 to about 1000nM (about 5 to about 80 µg/ml when the chsp60 is used), preferably about 200 to about 400nM, can be mentioned. The incubation can be performed using a culture vessel for animal cell cultivation; for example, 96-well plates and the like are preferably used. Cell density is exemplified by about 5x10⁵ to about 1x10⁶ cells/ml. The incubation can, for example, be performed in a CO₂ incubator (e.g., 5% CO₂ 95% air) at about 30°C to about 40°C for about 40 to about 72 hours, preferably about 44 to about 50 hours.

On the other hand, when the chsp60 species is provided in the form of cells capable of producing the same, cells of an established cell line can be stimulated with the chsp60 species by incubating the cells of the established monocytic cell line in the presence of the cells. The amount added of the cells capable of producing the chsp60 species may be any amount, as long as it can provide a level of stimulation equivalent to that with the above-described concentration of the chsp60; when using naturally occurring Cp cells, examples of such amounts include about 5x10⁵ to about 1x10⁷ IFU (Inclusion-Forming Units)/ml, preferably about 1x10⁶ to about 1x10⁷ IFU (Inclusion-Forming Units)/ml. The incubation can be performed under the same conditions as above.

In another embodiment of the present invention, the cells producing a chsp60 species may be cells of an established monocytic cell line themselves. That is, the screening of the present invention can be performed by transforming cells of an established monocytic cell line as host cells with an expression vector comprising the DNA encoding the chsp60 or a partial peptide thereof according to the above-described method, and incubating the thus-obtained transformant under the same conditions as described above.

In this case, it is more preferable that the transformant has been treated to make it capable of producing a chsp60 species only under predetermined conditions. As examples of the method of preparing such a transformant, a method using an expression vector wherein the DNA encoding the chsp60 or a partial peptide thereof is placed under the control of an inducible promoter (e.g., metallothionein promoter, heat shock promoters and the like), a method using the CRE-loxP system, and the like can be mentioned. When such a transformant is used, "stimulation with a chsp60 species" refers to loading with an inducer (or inducing stress), and "without stimulation" refers to non-induction of chsp60 species expression.

The test compound subjected to the screening of the present invention is not subject to limitation; for example, a compound library prepared using combinatorial chemistry technology, a random peptide library prepared by solid phase synthesis or the phage display method, or naturally occurring ingredients derived from microorganisms, animals, plants, marine organisms and the like, and the like can be mentioned.

The method of measuring the expression of the MMP-9 gene in cells of an established monocytic cell line is also not subject to limitation. For example, the gene expression amount can be measured at the RNA level by performing Northern hybridization using a nucleic acid comprising the MMP-9-encoding base sequence or a portion thereof as a probe with an RNA extracted from the cells of the established cell line as a template, or by performing quantitative RT-PCR using a synthetic oligo-DNA comprising a portion of the MMP-9-encoding base sequence as a primer with an RNA extracted from the cells of the established cell line as a template.

As examples of the nucleic acid comprising the MMP-9-encoding base sequence or a portion thereof, a nucleic acid comprising the base sequence shown by SEQ ID NO:3 or a base sequence capable of hybridizing to the base sequence under highly stringent conditions can be mentioned. The nucleic acid may be a DNA or an RNA, and may be a DNA/RNA chimera. Also, the nucleic acid may be single stranded or double stranded; when the nucleic acid is single stranded, it may be a sense strand or an antisense strand. When the nucleic acid is double stranded, it may be a DNA:RNA hybrid.

Alternatively, the expression of the MMP-9 gene can also be performed at a protein level using an anti-MMP-9 antibody by a commonly used immunoassay system. The anti-MMP-9 antibody used here may be a monoclonal antibody or a polyclonal antibody; in addition to the complete antibody molecule, the F(ab')₂, Fab' and Fab fractions thereof and the like may also be used.

Monoclonal antibodies or polyclonal antibodies against MMP-9 are commercially available (e.g., those produced by Amersham Company), or can be produced using MMP-9 or a partial peptide thereof or a salt thereof (hereinafter sometimes referred to as "an MMP-9 species (MMP-9s)") as an antigen according to an antibody or antiserum production method known per se. The MMP-9 protein used as an antigen is preferably, but is not limited to, a protein of the same derivation as the cells of the established monocytic cell line used in the screening. For example, when cells of an established human-derived monocytic cell line are used, a 92-kDa proprotein comprising the amino acid sequence shown by amino acid Nos. -87 to 601 in the amino acid sequence shown by SEQ ID NO:4, or an 88-kDa active protein comprising the amino acid sequence shown by amino acid Nos. 1 to 601, can be mentioned. An MMP-9 species can be obtained by purification from MMP-9-producing cells (e.g., mammal-derived monocytes, macrophages and the like) using a known technique of protein separation in the same manner as with a chsp60 species, by chemical synthesis by the solid phase synthesis method based on the amino acid sequence information shown by SEQ ID NO:4, by recombinant production using the DNA encoding MMP-9 cloned on the basis of the base sequence information shown by SEQ ID NO:3, or by cell-free synthesis with the MMP-9-encoding nucleic acid as a template.

A monoclonal antibody and polyclonal antibody against MMP-9 can, for example, be prepared as described below.

### [Preparation of monoclonal antibody]

### (a) Preparation of cells producing a monoclonal antibody

An MMP-9 species is administered as is, or along with a carrier or a diluent, to a warm-blooded animal at a site enabling antibody production by its administration. In order to increase antibody productivity upon the administration, Freund's complete adjuvant or Freund's incomplete adjuvant may be administered. Dosing is normally performed about two to 10 times in total every 2 to 6 weeks. As examples of the warm-blooded animal used, mammals such as monkeys, rabbits, dogs, guinea pigs, mice, rats, sheep, and goat (and non-mammal animals such as chicken) can be mentioned, with preference given to mice and rats.

For example, a hybridoma that produces a monoclonal antibody can be prepared by selecting an individual showing an antibody titer from among warm-blooded animals, e.g., mice, immunized with an antigen, collecting the spleen or lymph node 2 to 5 days after final immunization, and fusing antibody-producing cells contained therein with myeloma cells of the same animal species or of a different animal species. A measurement of antibody titer in an antiserum can be performed by, for example, reacting a protein labeled as described below with the antiserum, then determining the activity of the labeling agent bound to the antibody. The fusion can be performed according to a known method, for example, the method of Koehler and Milstein [Nature, 256, 495 (1975)]. As examples of the fusogen, polyethylene glycol (PEG), Sendai virus and the like can be mentioned, with preference given to PEG.

As examples of the myeloma cells, warm-blooded animal myeloma cells such as NS-1, P3U1, SP2/0, and AP-1 can be mentioned, with preference given to P3U1. The ratio by number of antibody-producing cells (splenocytes) and myeloma cells used is preferably about 1:1 to 20:1; cell fusion can be efficiently performed by adding PEG (preferably PEG1000 to PEG6000) at a concentration of about 10% to 80%, and incubating the cells at 20°C to 40°C, preferably 30°C to 37°C, for 1 to 10 minutes.

A hybridoma that produces a monoclonal antibody can be screened by, for example, a method that comprises adding a hybridoma culture supernatant to a solid phase (e.g., microplate) having a protein antigen adsorbed thereto, directly or along with a carrier, then adding an anti-immunoglobulin antibody (an anti-mouse immunoglobulin antibody is used when mouse cells are used for the cell fusion) labeled with a radioactive substance, an enzyme or the like, or Protein A, and detecting the monoclonal antibody bound to the solid phase; a method that comprises adding a hybridoma culture supernatant to a solid phase having an anti-immunoglobulin antibody or Protein A adsorbed thereto, adding a protein labeled with a radioactive substance, an enzyme or the like, and detecting the monoclonal antibody bound to the solid phase; and the like.

A monoclonal antibody can be selected according to a method known per se or a method based thereon. This selection of a monoclonal antibody can normally be achieved using a medium for animal cells supplemented with HAT (hypoxanthine, aminopterin, thymidine) and the like. Any selection and breeding medium for a monoclonal antibody can be used, as long as it allows the hybridoma to grow. For example, an RPMI 1640 medium containing 1% to 20%, preferably 10% to 20%, fetal calf serum, a GIT medium (Wako Pure Chemical Industries, Ltd.) containing 1% to 10% fetal calf serum, a serum-free medium for hybridoma culture (SFM-101, Nissui Seiyaku Co., Ltd.) and the like can be used. Cultivation temperature is normally 20°C to 40°C, preferably about 37°C. Cultivation time is normally 5 days to 3 weeks, preferably 1 week to 2 weeks. The cultivation may be performed normally in the presence of 5% gaseous carbon dioxide. The antibody titer of the hybridoma culture supernatant can be determined in the same manner as the above-described determination of antibody titer in antiserum.

The monoclonal antibody thus obtained can be separated and purified according to a method known per se, for example, a method of immunoglobulin separation and purification [e.g., salting-out, alcohol precipitation, isoelectric point precipitation, electrophoresis, adsorption and desorption with ion exchangers (e.g., DEAE), ultracentrifugation, gel filtration, specific purification comprising collecting only the antibody using an activated adsorbent such as an antigen-bound solid phase, Protein A, or Protein G, and dissociating the linkage to separate the desired antibody].

### [Preparation of polyclonal antibody]

A polyclonal antibody against MMP-9 can be produced according to a method known per se. For example, the polyclonal antibody can be produced by forming an immunoantigen (protein antigen) or a complex thereof with a carrier protein, immunizing a warm-blooded animal in the same manner as the above-described method of monoclonal antibody production, collecting a product containing an antibody against MMP-9 from the immunized animal, and separating and purifying the antibody.

Regarding the complex of immunoantigen and carrier protein used to immunize a warm-blooded animal, the kind of carrier protein and the mixing ratio of carrier and hapten may be any ones whatever they are, as long as an antibody against the immunizing hapten crosslinked to the carrier is efficiently produced; for example, a method that comprises coupling bovine serum albumin, bovine thyroglobulin, hemocyanin or the like to the hapten in a ratio by weight of 0.1 to 20, preferably 1 to 5, to 1 of hapten, can be used.

Various condensing agents can be used for the coupling of hapten and carrier; for example, glutaraldehyde, carbodiimide, maleimide activated ester, activated ester reagents comprising the thiol group or the dithiopyridyl group, and the like can be used.

The condensation product is administered as is, or along with a carrier or a diluent, to a warm-blooded animal at a site enabling antibody production by its administration. In order to increase antibody productivity upon the administration, Freund's complete adjuvant or Freund's incomplete adjuvant may be administered. Dosing is normally performed about three to 10 times in total every 2 to 6 weeks.

The polyclonal antibody can be collected from blood, ascites fluid and the like, preferably blood, of a warm-blooded animal immunized by the above-described method.

Polyclonal antibody titer in antiserum can be determined in the same manner as the above-described determination of antibody titer in serum. Separation and purification of the polyclonal antibody can be performed according to a method of immunoglobulin separation and purification as with the above-described separation and purification of monoclonal antibody.

The method of quantifying MMP-9 or a salt thereof using an anti-MMP-9 antibody is not to be subject to limitation; any assay method can be used, as long as the amount of antibody, antigen, or antibody-antigen complex corresponding to the amount of antigen in a test liquid is detected by a chemical or physical means and calculated from a standard curve generated using standard solutions containing known amounts of antigen. For example, nephelometry, the competitive method, the immunometric method, and the sandwich method are advantageously used, with preference given to the sandwich method described below, in terms of sensitivity and specificity.

Specifically, as the immunoassay method using an anti-MMP-9 antibody,
(i) a method comprising quantifying MMP-9 or a salt thereof in a test liquid (culture supernatant after incubation) by competitively reacting an anti-MMP-9 antibody and the test liquid and a labeled MMP-9 species, and measuring the ratio of the labeled MMP-9 bound to the antibody (competitive method), and
(ii) a method comprising quantifying MMP-9 or a salt thereof in a test liquid by simultaneously or sequentially reacting the test liquid and an anti-MMP-9 antibody insolubilized on a carrier and another labeled anti-MMP-9 antibody, and then measuring the activity of the labeling agent on the insolubilizing carrier (sandwich method), and the like can be mentioned.

In the quantification (ii) above, when one antibody recognizes the N-terminal portion of MMP-9, it is desirable that the other antibody be an antibody that recognizes another portion of MMP-9, for example, the C-terminal portion.

As examples of the labeling agent used for the assay method using a labeled substance, radioisotopes, enzymes, fluorescent substances, luminescent substances and the like can be mentioned. As examples of the radioisotopes used, [¹²⁵I], [¹³¹I], [³H], [¹⁴C] and the like can be mentioned. As examples of the enzymes, stable enzymes of high specific activity are preferred; for example, β-galactosidase, β-glucosidase, alkaline phosphatase, peroxidase, malate dehydrogenase and the like can be mentioned. As examples of the fluorescent substances, fluorescamine, fluorescein isothiocyanate and the like can be mentioned. As examples of the luminescent substances, luminol, luminol derivatives, luciferin, lucigenin and the like can be mentioned. Furthermore, the biotin-(strepto)avidin system may also be used for the binding of an antibody or antigen and the labeling agent.

For insolubilization of the antigen or antibody, physical adsorption may be used, and methods based on chemical binding, which are normally used to insolubilize or immobilize proteins, may also be used. As examples of the carrier, insoluble polysaccharides such as agarose, dextran and cellulose; synthetic resins such as polystyrene, polyacrylamide and silicone; glass and the like can be mentioned.

In the sandwich method, the insolubilized anti-MMP-9 antibody is reacted with a test liquid (primary reaction), and further reacted with another labeled anti-MMP-9 antibody (secondary reaction), and the activity of the labeling agent on the insolubilizing carrier is then measured, whereby the amount of MMP-9 or a salt thereof in the test liquid can be quantified. The primary and secondary reactions may be performed in the reverse order, and may be performed simultaneously or at a time interval. The labeling agent and the method of insolubilization may be the same as those described above. In immunoassays by the sandwich method, the antibody used for an immobilized antibody or a labeled antibody need not always be one kind; a mixture of two or more kinds of antibodies may be used to increase assay sensitivity.

In assaying MMP-9 or a salt thereof by the above-described sandwich method, the anti-MMP-9 antibodies used for the primary and secondary reactions are preferably antibodies having mutually different sites for binding with MMP-9. That is, regarding the antibodies used for the primary and secondary reactions, for example, when the antibody used for the secondary reaction recognizes the C-terminal portion of MMP-9, the antibody used for the primary reaction is preferably an antibody that recognizes a portion other than the C-terminal portion, for example, the N-terminal portion.

An anti-MMP-9 antibody can be used for assay systems other than the sandwich method, for example, the competitive method, the immunometric method, nephelometry and the like.

In the competitive method, an antigen in a test liquid and a labeled antigen are competitively reacted with an antibody, then the unreacted labeled antigen (F) and the labeled antigen bound to the antibody (B) are separated (B/F separation); the amount labeled in either B or F is measured to quantify the amount of antigen in the test liquid. For this reaction, the liquid phase method, which uses a soluble antibody as the antibody and uses polyethylene glycol, a secondary antibody against the above-described antibody (primary antibody) and the like for B/F separation, and the immobilization method, which uses a solid-immobilized primary antibody or uses a soluble primary antibody and a solid-immobilized secondary antibody, are available. In the liquid phase method, a homogenous system immunoassay such as surface plasmon resonance, fluorescence polarization immunoassay, fluorescence quenching immunoassay, or fluorescence energy transfer immunoassay can be used.

In the immunometric method, an antigen in a test liquid and an immobilized antigen are competitively reacted with a given amount of labeled antibody, and thereafter the solid phase is separated from the liquid phase, or an antigen in a test liquid is reacted with an excess amount of labeled antibody, an immobilized antigen is then added to bind the unreacted labeled antibody to the solid phase, and the solid phase is separated from the liquid phase. Subsequently, the amount of labeled antibody in either phase is measured to quantify the amount of antigen in the test liquid.

In nephelometry, the amount of insoluble precipitate resulting from the antigen-antibody reaction within the gel or in the solution is measured. Even when the amount of antigen in the test liquid is small and only a small amount of precipitate is obtained, laser nephelometry, which is based on laser scattering, and the like can be used advantageously.

In applying these individual immunoassays to the quantification method of the present invention, no special conditions, procedures or the like need to be set forth. The assay system for MMP-9 may be constructed with ordinary technical considerations of those skilled in the art on ordinary conditions and procedures in the respective methods. For details of these general techniques, reference can be made to reviews, texts and the like.

For example, Hiroshi Irie, ed., "Radioimmunoassay" (Kodansha Ltd., published in 1974), Hiroshi Irie, ed., "Sequel to the Radioimmunoassay" (Kodansha Ltd., published in 1979), Eiji Ishikawa et al., ed., "Enzyme Immonoassay" (Igakushoin, published in 1978), Eiji Ishikawa et al., ed., "Enzyme Immonoassay" (2nd ed.) (Igakushoin, published in 1982), Eiji Ishikawa et al., ed., "Enzyme Immonoassay" (3rd ed.) (Igakushoin, published in 1987), Methods in ENZYMOLOGY, Vol. 70 (Immunochemical Techniques (Part A)), ibidem, Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibidem, Vol. 92 (Immunochemical Techniques (Part E: Monoclonal Antibodies and General Immunoassay Methods)), ibidem, Vol. 121 (Immunochemical Techniques (Part I: Hybridoma Technology and Monoclonal Antibodies)) (all published by Academic Press Publishing) and the like can be referenced to.

Alternatively, the expression of the MMP-9 gene can also be measured with the enzyme activity of the resulting MMP-9 or a salt thereof as an indicator. Although the method of measuring the enzyme activity is not subject to limitation, for example, a method comprising subjecting a test liquid (culture supernatant after completion of incubation) to electrophoresis on a gel (e.g., SDS-polyacrylamide gel and the like) comprising a substrate for MMP-9 (e.g., gelatin, type IV collagen and the like), removing the SDS to refold the MMP-9, then incubating the gel in an appropriate buffer, and quantifying the density of the lytic band by image analysis, and the like can be mentioned.

Particularly preferably, the method of measuring the expression of the MMP-9 gene in the screening method of the present invention is the competitive ELISA method comprising competitively reacting a test liquid and an enzymatically labeled MMP-9 species to an anti-MMP-9 antibody.

As a result of the above-described measurement of the expression of the MMP-9 gene, for example, a test compound that decreases the expression amount of the MMP-9 gene with chsp60 stimulation by about 20% or more, preferably 30% or more, more preferably about 50% or more, can be selected as a compound that inhibits the induction of MMP-9 expression by the chsp60.

A compound that inhibits the induction of MMP-9 expression selected as described above is effective in the treatment of arteriosclerosis and/or the prevention/treatment of complications thereof, because it is capable of inhibiting the increase in MMP-9 secretion from foam cells derived from macrophages or vascular smooth muscle cells due to Cp infection to suppress the weakening of the fibrous coat of plaques, and hence to prevent plaque destabilization and collapse.

The present invention also provides a kit suitable for performing the above-described screening method. The screening kit of the present invention comprises cells of an established monocytic cell line and a chsp60 species or cells capable of producing the same or an expression vector comprising the DNA encoding the same. As the cells of the established monocytic cell line and the chsp60 species or the cells capable of producing the same or the expression vector comprising the DNA encoding the same, those mentioned above in the description of the screening method of the present invention can be used.

In an embodiment, the screening kit of the present invention comprises cells of an established monocytic cell line manipulated to be capable of producing a chsp60 species. As such cells of an established monocytic cell line, those mentioned above in the description of the screening method of the present invention can be used.

The screening kit of the present invention may further comprise a reagent for measuring the expression of the MMP-9 gene. For example, when the expression of the MMP-9 gene is measured using RT-PCR, the kit may further comprise, for example, a sense and an antisense primer capable of amplifying a fragment of MMP-9 RNA. Examples of the base sequence of the primer pair include, but are not limited to, the primer described in the above-mentioned Vehmaan-Kreula, P. et al., Arterioscler. Thromb. Vasc. Biol. (USA), 2001, Vol. 21, pp. e1-e8 and the like; those skilled in the art are able to easily construct another advantageous primer on the basis of the base sequence of the MMP-9-encoding nucleic acid (for example, the base sequence shown by SEQ ID NO:3), using as necessary a known primer design support software.

When the expression of the MMP-9 gene is measured using the competitive ELISA method, the kit may further comprise an anti-MMP-9 antibody and enzymatically labeled MMP-9 or a partial peptide thereof or a salt thereof. Here, as the anti-MMP-9 antibody and the enzymatically labeled MMP-9 species, those mentioned above in the description of the screening method of the present invention can be used respectively. The kit may further comprise a secondary antibody capable of specifically recognizing an anti-MMP-9 antibody (primary antibody) (for example, goat-derived anti-rabbit IgG antiserum and the like when the primary antibody is a rabbit-derived anti-MMP-9 IgG antibody) and a carrier for immobilizing the anti-MMP-9 antibody or the secondary antibody (e.g., microtiter plates, glass beads and the like).

The present invention also provides a therapeutic agent for arteriosclerosis and/or a prophylactic/therapeutic agent for complications thereof, comprising a substance capable of suppressing the induction of the expression of the MMP-9 gene by chsp60 in mammalian cells. The prophylactic/therapeutic agent can comprise a pharmacologically acceptable carrier as necessary.

As the "pharmacologically acceptable carrier", various organic or inorganic carrier substances in common use as pharmaceutical materials can be used, which are formulated as excipients, lubricants, binders, and disintegrants in solid preparations; as solvents, solubilizers, suspending agents, isotonizing agents, buffers, soothing agents and the like in liquid preparations. Pharmaceutical additives such as antiseptics, antioxidants, colorants, and sweeteners can also be used as necessary.

As examples of preferable excipients, lactose, sucrose, D-mannitol, D-sorbitol, starch, α-starch, dextrin, crystalline cellulose, low-substituted hydroxypropylcellulose, carboxymethylcellulose sodium, gum arabic, dextrin, pullulan, light silicic anhydride, synthetic aluminum silicate, magnesium metasilicate aluminate and the like can be mentioned.

As examples of preferable lubricants, magnesium stearate, calcium stearate, talc, colloidal silica and the like can be mentioned.

As examples of preferable binders, α-starch, cane sugar, gelatin, gum arabic, methylcellulose, carboxymethylcellulose, carboxymethylcellulose sodium, crystalline cellulose, sucrose, D-mannitol, trehalose, dextrin, pullulan, hydroxypropylcellulose, hydroxypropylmethylcellulose, polyvinylpyrrolidone and the like can be mentioned.

As examples of preferable disintegrants, lactose, sucrose, starch, carboxymethylcellulose, carboxymethylcellulose calcium, crosscarmellose sodium, carboxymethylstarch sodium, light silicic anhydride, low-substituted hydroxypropylcellulose and the like can be mentioned.

As examples of preferable solvents, water for injection, physiological saline, Ringer's solution, alcohol, propylene glycol, polyethylene glycol, sesame oil, corn oil, olive oil, cottonseed oil and the like can be mentioned.

As examples of preferable solubilizers, polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like can be mentioned.

As examples of preferable suspending agents, surfactants such as stearyltriethanolamine, sodium lauryl sulfate, laurylaminopropionic acid, lecithin, benzalkonium chloride, benzethonium chloride, and glyceryl monostearate; hydrophilic polymers such as polyvinyl alcohol, polyvinylpyrrolidone, carboxymethylcellulose sodium, methylcellulose, hydroxymethylcellulose, hydroxyethylcellulose, and hydroxypropylcellulose; polysorbates, polyoxyethylene hydrogenated castor oil and the like can be mentioned.

As examples of preferable isotonizing agents, sodium chloride, glycerin, D-mannitol, D-sorbitol, glucose and the like can be mentioned.

As examples of preferable buffers, buffer solutions such as of phosphates, acetates, carbonates and citrates, and the like can be mentioned.

As examples of preferable soothing agents, benzyl alcohol and the like can be mentioned.

As examples of preferable antiseptic agents, para-oxybenzoic acid esters, chlorobutanol, benzyl alcohol, phenethyl alcohol, dehydroacetic acid, sorbic acid and the like can be mentioned.

As examples of preferable antioxidants, sulfites, ascorbates and the like can be mentioned.

As examples of preferable colorants, water-soluble food tar colors (e.g., food colors such as Food Color Red No.2 and No.3, Food Color Yellow No. 4 and No.5, and Food Color Blue No. 1 and No.2), water-insoluble lake colors (e.g., aluminum salts of the aforementioned water-soluble food tar colors, and the like), natural colors (e.g., β-carotene, chlorophyll, red iron oxide and the like) and the like can be mentioned.

As examples of preferable sweeteners, saccharin sodium, dipotassium glycyrrhizinate, aspartame, stevia and the like can be mentioned.

As examples of dosage forms for the aforementioned pharmaceutical composition, oral preparations such as tablets, capsules (including soft capsules and microcapsules), granules, powders, syrups, emulsions, and suspensions; and non-oral preparations such as injections (e.g., subcutaneous injections, intravenous injections, intramuscular injections, intraperitoneal injections and the like), external preparations (e.g., nasal preparations, transdermal preparations, ointments and the like), suppositories (e.g., rectal suppositories, vaginal suppositories and the like), pellets, drip infusions, and sustained-release preparations (e.g., sustained-release microcapsules and the like) can be mentioned, and these can be safely administered orally or non-orally, respectively.

These pharmaceutical compositions can be produced by a method in common use in the field of drug formulation technology, for example, methods described in the Japanese Pharmacopoeia and the like. Specific methods of preparing preparations are described in detail below. The content of a compound obtained by the screening method of the present invention in the pharmaceutical composition varies depending on dosage form, dose of the compound and the like, and is, for example, about 0.1% to 100% by weight.

For example, an oral preparation is produced by adding an excipient (e.g., lactose, sucrose, starch, D-mannitol and the like), a disintegrant (e.g., carboxymethylcellulose calcium and the like), a binder (e.g., α-starch, gum arabic, carboxymethylcellulose, hydroxypropylcellulose, polyvinylpyrrolidone and the like), a lubricant (e.g., talc, magnesium stearate, polyethylene glycol 6000 and the like) and the like, to active ingredients, and compressing them, with coating performed, if necessary, using a coating base for the purpose of taste masking, enteric dissolution or sustained release, by a method known per se.

As examples of the coating base, sugar-coating bases, water-soluble film-coating bases, enteric film-coating bases, sustained-release film-coating bases and the like can be mentioned.

As the sugar-coating base, sucrose is used, which may be used in combination with one or two or more kinds selected from among talc, precipitated calcium carbonate, gelatin, gum arabic, pullulan, carnauba wax and the like.

As examples of the water-soluble film-coating base, cellulose polymers such as hydroxypropylcellulose, hydroxypropylmethylcellulose, hydroxyethylcellulose and methylhydroxyethylcellulose; synthetic polymers such as polyvinylacetal diethylaminoacetate, aminoalkylmethacrylate copolymer E [Eudragit E (trade name), Roehm Pharma GmbH], and polyvinylpyrrolidone; polysaccharides such as pullulan; and the like can be mentioned.

As examples of the enteric film-coating base, cellulose polymers such as hydroxypropylmethylcellulose phthalate, hydroxypropylmethylcellulose acetate succinate, carboxymethylethylcellulose, and cellulose acetate phthalate; acrylate polymers such as methacrylate copolymer L [Eudragit L (trade name), Roehm Pharma GmbH], methacrylate copolymer LD [Eudragit L-30D55 (trade name), Roehm Pharma GmbH], and methacrylate copolymer S [Eudragit S (trade name), Roehm Pharma GmbH]; naturally occurring substances such as shellac; and the like can be mentioned.

As examples of the sustained-release film-coating base, cellulose polymers such as ethylcellulose; acrylate polymers such as aminoalkylmethacrylate copolymer RS [Eudragit RS (trade name), Roehm Pharma GmbH] and ethyl acrylate/methyl methacrylate copolymer suspension [Eudragit NE (trade name), Roehm Pharma GmbH]; and the like can be mentioned.

The above-described coating bases may be used in a suitable mixture of two or more kinds thereof. Also, a light-blocking agent such as titanium oxide or iron sesquioxide may be used during coating.

An injection is produced by dissolving, suspending or emulsifying active ingredients, along with a dispersing agent (e.g., polysorbate 80, polyoxyethylene hydrogenated castor oil 60, polyethylene glycol, carboxymethylcellulose, sodium alginate and the like), a preservative (e.g., methylparaben, propylparaben, benzyl alcohol, chlorobutanol, phenol and the like), an isotonizing agent (e.g., sodium chloride, glycerin, D-mannitol, D-sorbitol, glucose and the like) and the like, in an aqueous solvent (e.g., distilled water, physiological saline, Ringer's solution and the like) or an oily solvent (e.g., vegetable oils such as olive oil, sesame oil, cottonseed oil and corn oil, propylene glycol and the like). If desired, additives such as a solubilizer (e.g., sodium salicylate, sodium acetate and the like), a stabilizer (e.g., human serum albumin and the like), and a soothing agent (e.g., benzyl alcohol and the like) may be used. The injection is usually filled in suitable ampoules.

Because the preparation thus obtained is safe and less toxic, it can be administered orally or non-orally to, for example, mammals (for example, humans, mice, rats, rabbits, sheep, swine, cattle, horses, cats, dogs, monkeys, chimpanzees and the like).

The dose of the prophylactic/therapeutic agent varies depending on target disease, seriousness, subject of administration, route of administration and the like; for example, in an adult patient with atherosclerosis (assuming a 60 kg body weight), the dose is about 0.1 to about 100 mg, preferably about 1.0 to about 50 mg, and more preferably about 1.0 to about 20 mg, per day, based on the amount of the active ingredient substance that suppresses the induction of MMP-9 expression, and this amount can be administered in a single dose or two or more divided doses.

Abbreviations for bases, amino acids and the like used in the present description are based on abbreviations specified by the IUPAC-IUB Commission on Biochemical Nomenclature or abbreviations in common use in relevant fields; some examples are given below. When an optical isomer may be present in amino acid, it is of the L-configuration, unless otherwise stated.
- DNA:: deoxyribonucleic acid
- cDNA:: complementary deoxyribonucleic acid
- A:: adenine
- T:: thymine
- G:: guanine
- C:: cytosine
- RNA:: ribonucleic acid
- mRNA:: messenger ribonucleic acid
- dATP:: deoxyadenosine triphosphate
- dTTP:: deoxythymidine triphosphate
- dGTP:: deoxyguanosine triphosphate
- dCTP:: deoxycytidine triphosphate
- ATP:: adenosine triphosphate
- EDTA:: ethylenediaminetetraacetic acid
- SDS:: sodium dodecyl sulfate
- Gly:: glycine
- Ala:: alanine
- Val:: valine
- Leu:: leucine
- Ile:: isoleucine
- Ser:: serine
- Thr:: threonine
- Cys:: cysteine
- Met:: methionine
- Glu:: glutamic acid
- Asp:: aspartic acid
- Lys:: lysine
- Arg:: arginine
- His:: histidine
- Phe:: phenylalanine
- Tyr:: tyrosine
- Trp:: tryptophan
- Pro:: proline
- Asn:: asparagine
- GIn:: glutamine
- pGlu:: pyrroglutamic acid
- Sec:: selenocysteine

The sequence identification numbers in the sequence listing of the present description show the following sequences.
[SEQ ID NO:1]
   This shows the base sequence of the DNA that encodes the chsp60 derived from the *Chlamydia pneumoniae* KKpn-1 strain.
[SEQ ID NO:2]
   This shows the amino acid sequence of the chsp60 derived from the *Chlamydia pneumoniae* KKpn-1 strain.
[SEQ ID NO:3]
   This shows the base sequence of the DNA that encodes human MMP-9 (preproprotein).
[SEQ ID NO:4]
   This shows the amino acid sequence of human MMP-9 (preproprotein).
[SEQ ID NO:5]
   This shows the base sequence of an oligonucleotide designed to function as a primer for amplifying the DNA that encodes the chsp60 derived from the *Chlamydia pneumoniae* KKpn-1 strain.
[SEQ ID NO:6]
   This shows the base sequence of an oligonucleotide designed to function as a primer for amplifying the DNA that encodes the chsp60 derived from the *Chlamydia pneumoniae* KKpn-1 strain.

### Examples

The present invention is hereinafter described in more detail by means of the following reference examples and working examples. These examples, however, are given only for the sake of exemplification, and not to limit the scope of the present invention.

Note that the gene manipulation procedures described below were performed according to methods described in a book (Maniatis et al., Molecular Cloning, Cold Spring Harbor Laboratory, 1989), methods described in protocols attached to reagents, and the like.

### [Example 1]

### Reference Example 1: Cloning of the chsp60 gene

Cloning of the chsp60 gene was performed by a PCR method with a DNA extracted from Cp-infected cells as a template.

After a plate (25 cm² flask) inoculated with HEp-2 cells (about 2.5x10⁵ cells/ml; obtained from Dainippon Pharmaceutical Co., Ltd.) was cultured in a gaseous carbon dioxide incubator at 37°C overnight, the monolayer supernatant was discarded; a prepared bacterial liquid of the *Chlamydia pneumoniae* (Cp) KKpn-1 strain [Kishimoto T. and Soejima R., Intern. Med., 32: 934-937 (1993)] was inoculated to Iscove's Modified Dulbecco's Medium (IMDM) (supplemented with 10% FBS, 100 µg/mL streptomycin, 50 µg/mL gentamicin, and 0.5 µg/mL cycloheximide) to infect the HEp-2, and the Cp was cultured. After 72 hours of cultivation, the infected cells were recovered using a cell scraper, twice washed with PBS, then resuspended in 4 ml of TE buffer solution, and repeatedly frozen at -80°C and thawed in three cycles to disrupt the cells, thereafter this was treated with phenol and precipitated with ethanol, and the obtained nucleic acid fraction was used as a PCR template. Primers prepared with reference to the base sequence of the chsp60 (GroEL) gene derived from the Cp AR-39 strain (GenBank Accession NO. M69217) [5'-ggaaggcatatggcagcgaaaaatattaaata-3' (SEQ ID NO:5) and 5'-ggaaggcatatgctagtagtccattcctgcgctt-3' (SEQ ID NO:6)], 50 pmol each, were added to a PCR reaction liquid, and PCR was performed with KOD DNA Polymerase (Toyobo) using the Gene Amp PCR System 9700 (Applied Biosystems) (reaction conditions: 25 cycles of treatment at 95°C for 30 seconds, at 55°C for 30 seconds, and at 72°C for 1 minute).

After the DNA fragment amplified above was digested with the restriction endonuclease NdeI (Takara Shuzo), agarose gel electrophoresis was performed, and the fragment was recovered. The DNA fragment and the *Escherichia coli* expression plasmid pET21a (obtained from NOVAGEN), previously cleaved with NdeI, were mixed and joined using the DNA Ligation Kit Ver.2 (Takara Shuzo) to yield the plasmid pHSP21-1 for chsp60 expression. *Escherichia* coli BL21(DE3) competent cells (Toyobo) were transformed with this plasmid to yield the transformant *Escherichia* coli BL21(DE3)/pHSP21-1 strain. This *Escherichia* coli strain has been deposited with the International Patent Organism Depositary, National Institute of Advanced Industrial Science and Technology (Central 6, 1-1-1, Higashi, Tsukuba, Ibaraki, 305-8566 Japan) under the accession number of FERM BP-8430 since July 16, 2003].

### [Example 2]

### Reference Example 2: Preparation of recombinant chsp60

One loopful of the *Escherichia* coli BL21(DE3)/pHSP21-1 strain obtained in Reference Example 1 was inoculated to 1 L of a seed culture medium (1% Tryptone, 0.5% Yeast Extract, 0.5% NaCl and 50 mg/L ampicillin), and cultured at 30°C overnight for a seed culture. The entire quantity of this culture was transferred to 18.5 L of a main culture medium (1.2% Tryptone, 2.4% Yeast Extract, 0.2% Bactopeptone, 0.05% NaCl, 1.5% Glucose, 50 mg/L ampicillin and 50 mg/L Vitamin-B1), and main culture was initiated under the conditions of 37°C, a stirring rate of 150 rpm, an aeration rate of 20 NL/min, a dissolved oxygen concentration of 2.0 ppm, and a pH control level of 6.8 while measuring the Klett unit value over time. When the Klett unit value reached 800, Isopropyl β-D(-)-Thiogalactopyranoside (IPTG) was added to obtain a final concentration of 1mM, and cultivation was continued for another 4 hours to induce chsp60 expression, followed by centrifugation at 6000 rpm for 20 minutesto recover the expressing cells.

After 350 g of the obtained cells was suspended by the addition of 2.7 L of a 50mM Tris/HCl (pH 8.0) containing 5mM disodium ethylenediaminetetraacetate and 1mM (p-amidinophenyl)methanesulfonyl fluoride, the cells were disrupted at 1,000 to 1,100 bar using a high-pressure homogenizer (PANDA, NIRO SOAVI Company) with thorough cooling with ice. This disrupted cell liquid was centrifuged (17,700xG, 120 minutes, Beckman Instruments) to yield 3.15 L of a centrifugation supernatant. To the obtained centrifugation supernatant, 300 mL of 25(w/v)% streptomycin solution was added, followed by vigorous stirring; the resulting solution was allowed to stand at 4°C for 3 days and then centrifuged (12,227xG, 60 minutes, Beckman Instruments), to yield 3.6 L of a centrifugation supernatant. Subsequently, after this centrifugation supernatant was thoroughly dissolved by the addition of 1,062 g of ammonium sulfate, the solution was allowed to stand at 4°C overnight and then centrifuged (12,227xG, 60 minutes, Beckman Instruments), and the obtained precipitate was dissolved by the addition of 1.5 L of 20mM Tris/HCl (pH 8.0). After this solution was dialyzed against 10 L of 10mM Tris/HCl (pH 8.0), the internal dialyzate was further desalinized using ultrafiltration (PSU, 10k, SARTORIUS) to yield 3 L of a desalinized liquid. After the obtained desalinized liquid was passed through, and adsorbed to, a Toyopearl DEAE-650M column (11.3 cm diameter x 23 cm, Tosoh Corporation), previously equilibrated with 50mM Tris/HCl (pH 8.0), 50mM Tris/HCl (pH 8.0) and then a 50mM Tris/HCl (pH 8.0) containing 50mM sodium chloride were passed through the column to thoroughly wash the column. After the washing, a 50mM Tris/HCl (pH 8.0) containing 150mM sodium chloride was passed through the column to yield 5 L of an eluate containing the chsp60. This eluate was replaced with a 50mM Tris/HCl (pH 8.0) containing 2M urea and simultaneously concentrated using ultrafiltration (PSU, 10k, SARTORIUS) to yield 2 L of a concentrate. Subsequently, this concentrate was adsorbed to a DEAE-5PW column (10.5 cm diameter x 20 cm, Tosoh Corporation), previously equilibrated with a 50mM Tris/HCl (pH 8.0) containing 2M urea; after the column was thoroughly washed, solution B was passed through the column with a concentration gradient of 10 to 50% B (B=50mM Tris/HCl, 2M urea, 500mM sodium chloride, pH 8.0) at a flow rate of 50 mL/min for 80 minutes, to yield 2 L of a chsp60 eluate. After this eluate was desalinized using ultrafiltration (PSU, 10k, SARTORIUS ), the desalinized liquid was again adsorbed to a DEAE-5PW column (10.5 cm diameter x 20 cm, Tosoh Corporation), previously equilibrated with a 50mM Tris/HC1 (pH 8.0) containing 2M urea; after the column was thoroughly washed, solution B was passed through the column with a concentration gradient of 0 to 40% B (B=50mM Tris/HCl, 2M urea, 500mM sodium chloride, pH 8.0) at a flow rate of 50 mL/min for 80 minutes, to yield 555 mL of a chsp60 eluate. This eluate was passed through a Sephadex G-25 column (5 cm diameter x 50 cm, Amersham Biosciences K.K.), previously equilibrated with Dulbecco's phosphate-buffered saline, and 650 mL of the obtained chsp60 fraction was further passed through an Acticlean Etox column (5 cm diameter x 20 cm, Sterogene Company), previously equilibrated with Dulbecco's phosphate-buffered physiological saline, to remove endotoxins. Finally, 760 mL of the endotoxin-removed effluent was subjected to sterile filtration (Sterivex filter, Nihon Millipore Ltd.) to yield 760 mL of a chsp60 solution.

### [Example 3]

### Reference Example 3: Characteristic analysis of recombinant chsp60

### (a) Analysis using SDS polyacrylamide gel electrophoresis

To the purified chsp60 obtained in Reference Example 2, an equal amount of a sample buffer containing 100mM DTT [Laemmli, Nature, Vol. 227, p. 680 (1979)] was added; after vigorous stirring and 3 minutes of heating at 95°C, electrophoresis was performed using Perfect NT Gel 10-20% (DRC Company). As a result of Coomassie Brilliant Blue staining of the gel after electrophoresis, a single band was observed at about 60 kDa, confirming that the purified chsp60 was nearly a simple substance.

### (b) N-terminal amino acid sequence analysis

The N-terminal amino acid sequence of the purified chsp60 obtained in Reference Example 2 was determined using a gas-phase protein sequencer (Applied Biosystems Company, model 492 type). As a result, this sequence agreed with the N-terminal amino acid sequence of the HSP60 derived from the *Chlamydia pneumoniae* KKpn-1 strain deduced from the cDNA base sequence (SEQ D NO:1), except that the N-terminal amino acid Met was lacked from the obtained chsp60.

### [Example 4]

### Test Example 1: Changes over days in the induction of TNFα and MMP-9 production by chsp60 stimulation in cells of an established monocytic cell line

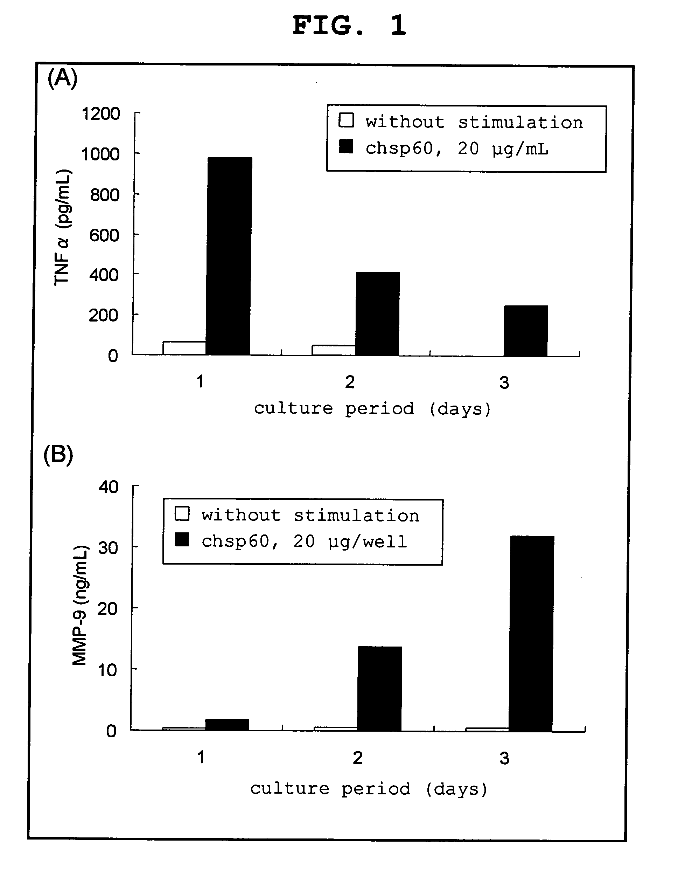

An established monocytic cell line THP-1 was purchased from the American Type Culture Collection (ATCC). The chsp60 prepared in Reference Example 2 was added to a 1x10⁶ cells/mL THP-1 cell suspension prepared with an RPMI 1640 medium supplemented with 1% fetal bovine serum (containing 100 µg/mL streptomycin, 100 units/mL penicillin and 50 µg/mL gentamicin) at 20 µg/mL, and this was cultured in ambient conditions of 5% CO₂ and 37°C. After 1 day, 2 days and 3 days of cultivation, each culture supernatant was collected, and the MMP-9 content in the supernatant was measured using an ELISA kit manufactured by Amersham Company and the TNFα content in the supernatant was measured using an ELISA kit manufactured by Genzyme Corporation. The culture supernatant of THP-1 cells cultured in the same manner, but without the addition of the chsp60, were used as control. These experiments were performed using 96-well plates with a liquid volume of 200 µL/well. The results are shown in Figure 1. The induction of TNFα production maximized at 1 day of cultivation and then decreased, whereas the induction of MMP-9 production increased over time during the cultivation period.

### [Example 5]

### Test Example 2: chsp60 concentration dependence of the induction of MMP-9 production in cells of an established monocytic cell line

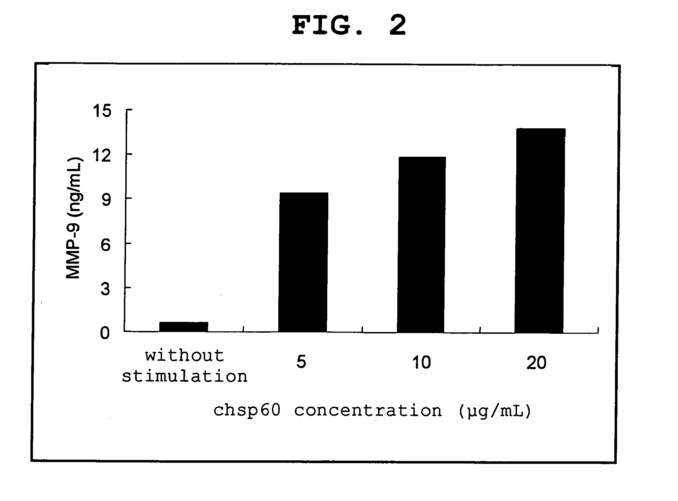

The chsp60 prepared in Reference Example 2 was added to a 1x10⁶ cells/mL THP-1 cell suspension prepared in the same manner as Example 1 at 5, 10 or 20 µg/mL, and this was cultured in ambient conditions of 5% CO₂ and 37°C. After 2 days of cultivation, the culture supernatant was collected, and the MMP-9 content in the supernatant was measured using an ELISA kit manufactured by Amersham Company. The culture supernatant of THP-1 cells cultured in the same manner, but without the addition of the chsp60, was used as control. The results are shown in Figure 2. The induction of MMP-9 production increased with dependence on chsp60 concentration.

### Industrial Applicability

The screening method of the present invention is advantageous in that higher levels of MMP-9 expression are induced by using cells of an established monocytic cell line than monocytes isolated from blood and primary macrophages.

The screening method of the present invention is effective as a highly sensitive screening system for a novel therapeutic drug for arteriosclerosis capable of suppressing the progression of arteriosclerosis due to infection with *Chlamydia pneumoniae* because higher levels of MMP-9 expression are induced by using cells of an established monocytic cell line than monocytes isolated from blood and primary macrophages.

### Free-text of Sequence Listing

SEQ ID NO:5
   An oligonucleotide designed to function as a primer for amplifying the DNA that encodes the chsp60 derived from the *Chlamydia pneumoniae* KKpn-1 strain.
SEQ ID NO:6
   An oligonucleotide designed to function as a primer for amplifying the DNA that encodes the chsp60 derived from the *Chlamydia pneumoniae* KKpn-1 strain.

This application is based on a patent application No. 2003-316572 filed September 9, 2003 in Japan, the contents of which are hereby incorporated by reference.

## Claims

1. A screening method for a therapeutic substance for arteriosclerosis and/or a prophylactic/therapeutic substance for complications thereof, which comprises using cells of an established monocytic cell line and a *Chlairiydia pneumoniae-*derived 60-kDa heat shock protein or a partial peptide thereof or a salt thereof or cells capable of producing the same (the cells may be identical to, or different from, the cells of the established monocytic cell line).

2. The screening method of claim 1, which comprises stimulating cells of an established monocytic cell line with a *Chlamydia pneumoniae*-derived 60-kDa heat shock protein or a partial peptide thereof or a salt thereof in the presence and absence of a test compound, and comparing the expressions of the matrix metalloproteinase-9 gene under the both conditions.

3. The screening method of claim 1, which comprises infecting cells of an established monocytic cell line with *Chlamydia pneumoniae* in the presence and absence of a test compound, and comparing the expressions of the matrix metalloproteinase-9 gene under the two conditions.

4. The screening method of claim 1, which comprises comparing the expressions of the matrix metalloproteinase-9 gene in cells of an established monocytic cell line capable of producing a *Chlamydia pneumoniae*-derived 60-kDa heat shock protein or a partial peptide thereof or a salt thereof in the presence and absence of a test compound.

5. The screening method of claim 1, which comprises using cells of an established monocytic cell line wherein the expression of the matrix metalloproteinase-9 gene with 48 hours of stimulation with 100nM of a *Chlamydia pneumoniae*-derived 60-kDa heat shock protein or a partial peptide thereof or a salt thereof is not less than 5 times the expression without the stimulation.

6. The screening method of claim 1, wherein the cells of the established monocytic cell line show substantially no expression of the matrix metalloproteinase-9 gene without stimulation.

7. The screening method of claim 1, wherein the cells of the established monocytic cell line are cells of an established cell line selected from the group consisting of THP-1, U-937, P31/FUJ, RAW264.7, and J774A.1.

8. The screening method of claim 1, wherein the cells of the established monocytic cell line are cells of the established THP-1 cell line.

9. A screening kit for a therapeutic substance for arteriosclerosis and/or a prophylactic/therapeutic substance for complications thereof, which comprises cells of an established monocytic cell line, and a *Chlamydia pneumoniae-*derived 60-kDa heat shock protein or a partial peptide thereof or a salt thereof or cells capable of producing the same (the cells may be identical to, or different from, the cells of the established monocytic cell line).

10. The screening kit of claim 9, wherein the cells capable of producing a *Chlamydia pneumoniae*-derived 60-kDa heat shock protein or a partial peptide thereof or a salt thereof are cells of *Chlamydia pneumoniae* or an established monocytic cell line.

11. The screening kit of claim 9, wherein the cells of the established monocytic cell line are cells of an established cell line selected from the group consisting of THP-1, U-937, P31/FUJ, RAW264.7 and J774A.1.

12. The screening kit of claim 9, wherein the cells of the established monocytic cell line are cells of the established THP-1 cell line.

13. A therapeutic agent for arteriosclerosis and/or a prophylactic/therapeutic agent for complications thereof, which comprises a substance capable of suppressing the induction of the expression of the matrix metalloproteinase-9 gene by a *Chlamydia pneumoniae*-derived 60-kDa heat shock protein in mammalian cells.

14. A therapeutic method for arteriosclerosis and/or a prophylactic or therapeutic method for complications thereof in a mammal, which comprises administering to the mammal an effective amount of a substance capable of suppressing the induction of the expression of the matrix metalloproteinase-9 gene by a *Chlamydia pneumoniae*-derived 60-kDa heat shock protein in mammalian cells.

15. A use of a substance capable of suppressing the induction of the expression of the matrix metalloproteinase-9 gene by a *Chlamydia pneumoniae*-derived 60-kDa heat shock protein in mammalian cells, for producing a therapeutic agent for arteriosclerosis and/or a prophylactic or therapeutic agent for complications thereof.
